# EUROPEAN PATENT SPECIFICATION

(11) **EP 3 148 613 B1**
(45) Date of publication and mention of the grant of the patent: **28.08.2019**
(21) Application number: 15724313.0
(22) Date of filing: 27.05.2015
(51) Int. Cl.: A61M 5/19, A61M 5/20, A61M 5/178

(54) **HOUSING FOR A DRUG DELIVERY DEVICE AND DRUG DELIVERY DEVICE**
GEHÄUSE FÜR EINE ARZNEIMITTELABGABEVORRICHTUNG UND ARZNEIMITTELABGABEVORRICHTUNG
CAPUCHON POUR DISPOSITIF D'ADMINISTRATION DE MÉDICAMENTS ET DISPOSITIF D'ADMINISTRATION DE MÉDICAMENTS

(30) Priority: 28.05.2014 EP 14170336
(43) Date of publication of application: 05.04.2017
(73) Proprietor: Sanofi-Aventis Deutschland GmbH, 65929 Frankfurt am Main (DE)
(72) Inventor: HOLTWICK, Marc, 65926 Frankfurt am Main (DE); MARX, Wolfgang, 65926 Frankfurt am Main (DE); BODE, Andreas, 65926 Frankfurt am Main (DE); WEST, James, Bristol Cheshire BS8 45Y (GB); HAYTON, Paul, St. Andrews Bristol BS6 5AU (GB); COOP, James, Clifton Bristol BS8 4ED (GB); KILBANE, Christopher, Bristol BS10 5ES (GB)
(74) Representative: Keil & Schaafhausen Patent- und Rechtsanwälte PartGmbB
(86) International application number: PCT/EP2015/061626
(87) International publication number: WO 2015/181191

(56) References cited:
- WO-A1-2013/079643
- WO-A2-94/22507
- US-A1- 2013 253 440

## Description

The invention is directed to a housing for a drug delivery device with a first receiving section for retaining a primary drug delivery assembly and a second receiving section for retaining a secondary drug delivery assembly. The present invention is also directed to drug delivery device comprising a respective housing.

Certain disease states require treatment using one or more different medicaments. Some drug compounds need to be delivered in a specific relationship with each other in order to deliver the optimum therapeutic dose. Here, combination therapy may be desirable, but not possible in a single formulation for reasons such as, but not limited to, stability, compromised therapeutic performance and toxicology.

For example, in some cases it might be beneficial to treat a diabetic with a long acting insulin and with a glucagon-like peptide-1 (GLP-1), which is derived from the transcription product of the proglucagon gene. GLP-1 is found in the body and is secreted by the intestinal L cell as a gut hormone. GLP-1 possesses several physiological properties that make it (and its analogs) a subject of intensive investigation as a potential treatment of diabetes mellitus. Another example of a medicament combination is the administration of a pain reliever in combination with a medicament for treating osteoarthritis.

Drug delivery devices of the aforementioned kind often have applications where regular injection by persons without formal medical training occurs. This is increasingly common among patients having diabetes or the like, e.g. osteoarthritis. Self-treatment enables such patients to conduct effective management of their disease.

In combination therapy, a primary medicament and a secondary medicament are delivered in a specific relationship to deliver the optimum therapeutic dose. The injection devices of the generic kind usually comprise a housing in which two or more drug delivery assemblies are retained. Such devices include a primary drug delivery assembly for dispensing the primary medicament such as the long-acting insulin and a secondary drug delivery assembly for dispensing the secondary medicament, such as GLP-1. Some kinds of drug delivery assemblies comprise a compartment such as a cartridge holder for accommodating a replaceable medicament container such as a cartridge which stores the medicament.

In some cases, depending on the patient or the stage of the therapy, an effective treatment requires variations in the quantities and/or proportions of the medicaments making up the combined therapy. For example, the patient may require a non-adjustable fixed dose of e.g. the secondary medicament in combination with an adjustable variable dose of the primary medicament.

WO 2012/072569 A1 discloses a drug delivery device with a housing retaining a first drug delivery device and a second drug delivery device. The drug delivery devices comprise a dose setting mechanism, each. A dose limiting system mechanically couples the dose setting mechanisms. The first dose setting mechanism includes a drive gear which is coupled a dose dial such that by rotating the dose dial, the drive gear in rotated in the same direction. The second dose setting mechanism includes a driven gear which constantly engages the drive gear and which engages a spindle by an internal thread. During dose setting, rotation of the first dose setter rotates the drive gear which forces the spindle of the second dose setting mechanism to move in axial direction by the threaded engagement with the driven gear. Axial travel of the spindle lifts a stopper that is threadedly engaged to the spindle. An increasing gap between the stopper and a second dose setter determines the maximum settable dose of the second medicament, which can be set by the user by manually displacing a dose button in axial direction until the proximal end of the dose button abuts the stopper.

WO 2012/072539 A1 discloses a drug delivery device and a method for sequentially delivering at least two medicaments. The device includes a rotationally driven variable dose setting mechanism operably connected to a first cartridge containing a first medicament and a fixed dose setting mechanism operably connected to a second cartridge containing a second medicament. A splined dose setter is connected to both setting mechanism. The dose setter is detachably connected to the fixed dose setting mechanism via a connecting feature such that proximal axial movement of the dose setter causes proximal movement of the fixed dose setting mechanism and setting of a fixed dose. After the fixed dose is set, the dose setter disengages from the connecting feature which in turn allows rotation of the splined dose setter to be rotated and the variable dose to be set. Once the variable dose is set, the splined dose setter is pushed in distal direction by what the variable dose is delivered. After the variable dose is delivered, the dose setter is reattached to the fixed dose mechanism and further distal movement of the dose setter causes the fixed dose to be delivered.

US 2013/0253440 A1 discloses also a drug delivery device with a housing retaining a first drug delivery device and a second drug delivery device. The drug delivery devices comprise a dose setting mechanism, each. The second dose setter is a button.

The effectiveness of a combined delivery of medicaments may require one or more doses to be delivered sequentially with one of the two medicaments being injected into the human body prior to the delivery of the other medicament. Such treatment may be conducted with devices that include two separate dispensing mechanisms that are actuated independently from each other such that the dispensing mechanisms are activated successively. This, however, may be hazardous for patients that are physically or mentally impaired or otherwise disadvantaged. It is desirable to have a device that is provided with merely one dispense button respectively an actuator which the patient can trigger and which leads to a sequential delivery of the primary and the secondary medicament. Known in the art are also electrical drug delivery devices, but these devices require an electrical energy source and are rather expensive and complex in their design. New developed drug delivery device also have to pass long-time approval procedure before competent authorities before they can be placed on the market, as setting mechanisms and the like have to fulfill strict requirements. In this regard, it would be favorable, especially for manufactures, to be able to fall back on drug delivery devices or drug delivery assemblies that have already been approved. Facing these challenges, there is a strong need for devices and device components for the delivery of two or more medicaments that are suitable for injecting different medication profiles in a single injection or delivery step. The setting of the doses and the injection procedure should be safe, simple, manageable and convenient for the user to perform.

It is an object of the invention to provide for a housing and a drug delivery device of the aforementioned kind which enables for convenient actuation of a drug delivery assembly retained in the housing. Further, safe dose setting shall be provided with a minimum of influences by the actuation mechanics. Another object of the invention is to provide for an individual setting of doses of two medicaments. Also, a drug delivery device shall be provided with improved safety and application capabilities.

The above problem is solved by a housing for a drug delivery device as defined in claim 1.

According to the invention, the housing comprises a first receiving section and a second receiving section. The first receiving section is configured for retaining a primary drug delivery assembly and the second receiving section is configured for retaining a secondary drug delivery assembly. The housing comprises an actuator arranged such that it is movable along a first longitudinal axis and comprising a first engagement section and a lever actuatable setting element located in the second receiving section and an actuation collar located in the second receiving section and comprising a second engagement section. The housing further comprises a setting element movable around a second longitudinal axis from a first position into a second position during a setting movement, wherein the setting element drives the actuation collar into engagement with the actuator during the setting movement. In the first position of the setting element the actuator is free to move relative to the actuation collar and in the second position of the setting element the actuation collar engages the actuator such that an actuation movement of the actuator is transferred to the actuation collar.

The actuateable setting element may be configured such that it can be manipulated from the outside of the housing by the user. The actuation collar may be movable between a non-engagement position relative to the actuator where the first engagement section and the second engagement section are not engaged and an engagement position relative to the actuator where the first engagement section and the second engagement section are engaged. The setting element may be adapted to the actuation collar such that the setting element moves the actuation collar from the non-engagement position with the actuator into the engagement position with the actuator when the setting element moves from the first position into the second position. For example, the setting element and the actuation collar may be provided with respective force transfer means such as abutment surfaces. The actuator may be slidably received in the housing. The housing may be provided with guiding means such as inner webs formed on inner surfaces of the housing and configured to guide the actuator during the actuation movement.

When the setting element is in the first position, the actuation collar is in the non-engagement position with the actuator and the actuator is free to move relative to the actuation collar, e.g. between a first and a second position. Accordingly, when the actuator is actuated by the user, the actuation movement is not transferred to the actuation collar and the actuation collar remains stationary in longitudinal direction, e.g. with respect to the second longitudinal axis. When the setting element is in the second position, the actuation collar is in the engagement position with the actuator. Accordingly, when the actuator is actuated by the user and moved from its first position to its second position, the actuation movement is transferred to the actuation collar such that the actuation collar is moved from a first position to a second position, e.g. from a first proximal position to a second distal position.

The setting movement of the setting element from the first into the second position results in the engagement between the actuation collar and the actuator such that an actuation of the actuator is transferred to the actuation collar.

A significant benefit of the present invention is that it couples the actuation means (actuator) provided by the housing for initiating the dispense of a medicament to the inner actuation collar. The setting element may be coupled to a secondary dose setting mechanism and the actuation collar may configured such as to actuate a secondary dose dispensing mechanism when the actuation movement is transferred to the actuation collar. The housing enables the user to set a dose of medicament in the secondary drug delivery assembly by directly manipulating the secondary dose setting mechanism via the setting element, which is safe in terms of setting accuracy. The setting movement comes along with a mechanical connection between the actuation collar and the actuator. The user may initiate the delivery of a medicament of a secondary drug delivery assembly retained in the second receiving section by conveniently using the actuator of the housing.

Correspondingly, the actuator can be used to initiate the delivery of a medicament of a primary drug delivery assembly retained in the first receiving section. Then, the actuation of two drug delivery assembly with only a single actuating element is possible. The user may directly set the secondary dose with the secondary dose setting mechanism independent from the actuation mechanism. By manipulating the setting element to set a dose in the secondary drug delivery assembly, the actuation collar is coupled to the actuator. With the setting movement, a mechanical connection for later actuation between the actuation collar in the second receiving section and the actuator is established. Accordingly, the invention may also provide for individual dose setting in the drug delivery assemblies and common dose delivery with a shared actuator, also.

The first and the second longitudinal axis may be aligned in a parallel relationship and preferably parallel to a longitudinal axis of the housing. First longitudinal axis or second longitudinal axis may also coincide with the longitudinal axis of the housing. Moreover, the first and the second longitudinal axis may extend from a proximal end of the housing to a distal end of the housing. The distal end of the housing may correspond to a dispensing end, such that actuation of a first and/or a second dispensing mechanism of a first and/or a second drug delivery assembly retained in the respective receiving section of the housing causes the respective medicament to flow in distal direction. Accordingly, the distal end may be provided for attachment to a dispense interface with an injection needle. An axis between the distal end and the proximal end of the housing may correspond to or may be parallel to the longitudinal axis of the housing. The proximal end of the housing may correspond to an actuation end, where the user may actuate the actuator, preferably by driving the actuator in distal direction.

The housing may be advantageously used in combination with a primary drug delivery assembly with a primary dose setting mechanism and a primary dose dispensing mechanism and a secondary drug delivery assembly with a secondary dose setting mechanism and a secondary dose dispensing mechanism. The setting element is preferably configured to be coupled to the secondary dose setting mechanism such that the setting movement of the setting element leads to the setting of a dose in the secondary drug delivery assembly. Preferably, the actuation collar is configured to actuate the secondary dose dispensing mechanism when actuation movement is transferred to the actuation collar. For that purpose, the actuation collar may be movable between two positions relative to the secondary dose dispensing mechanism or the secondary drug delivery assembly, wherein when the actuation collar is moved from a first into a second position, the actuation collar actuates the secondary dose dispensing mechanism such that the secondary medicament is delivered. Further, the actuator may be configured to actuate the primary dose dispensing mechanism by the actuation movement such that the primary medicament is delivered. The housing may be configured such that the user can manipulate the primary dose setting mechanism from the outside of the housing.

With the housing, the secondary dose setting mechanism can be directly and individually set by the user. Further, the primary dose setting mechanism and the secondary dose setting mechanism may be set independently from each other, wherein a mechanical connection for actuation resp. the dispensing step is automatically established by a setting movement. Mutual influence is effectively reduced. Furthermore, drug delivery assemblies may be received in the housing that have already passed approval procedures before the competent authorities, which is a benefit in terms of economic efficiency.

A further embodiment of the invention concerns a housing for a drug delivery device comprising a first receiving section for retaining a primary drug delivery assembly with a primary dose setting mechanism to set a dose of a primary medicament and a primary dose dispense mechanism to dispense a set dose of the primary medicament; and a second receiving section for retaining a secondary drug delivery assembly with a secondary dose setting mechanism to set a dose of a secondary medicament and a secondary dose dispense mechanism to dispense a set dose of the secondary medicament; an actuator arranged such that it is movable along a first longitudinal axis and comprising a first engagement section, wherein the actuator is configured to actuate the primary dose dispensing mechanism by an actuation movement such that the primary medicament is delivered; a preferably lever actuatable setting element located in the second receiving section and an actuation collar comprising a second engagement section, wherein the setting element is configured to be coupled to the secondary dose setting mechanism such that a (rotational) setting movement of the setting element around a second longitudinal axis from a first position into a second position leads to the setting of a dose in the secondary drug delivery assembly, wherein the actuation collar is configured to actuate the secondary dose dispensing mechanism when the actuation movement is transferred to the actuation collar such that the secondary medicament is delivered, and wherein the setting element drives the actuation collar into engagement with the actuator during the setting movement, wherein in the first position of the setting element the actuator is free to move relative to the actuation collar and in the second position of the setting element the actuation collar engages the actuator such that the actuation movement of the actuator is transferred to the actuation collar.

The first position of the setting element may correspond to an intial position of the setting element prior to setting a medicament dose. The second position may correspond to a final position of the setting element after the setting process, respectively after the medicament dose has been set. The setting movement of the setting element from the first into the second position may correspond to a predetermined set dose or fixed dose. For example, the first position may correspond to a set dose of zero units, which is the usual initial position before a dose is set by the patient. The second position may correspond to a set dose of 10 units. The actuation movement may take place after the setting process and may correspond to the action a patient conducts to inject the set dose of medicament. Such actuation movement is, for example, the displacement or the pressing of a dispense button.

The term "fixed dose" as used herein can be characterized as a dose value that is defined by the construction of the housing, the drug delivery assembly or the drug delivery device, wherein the user is only able to inject a specific dose. The user is not in the position to inject lower or higher doses of the medicament. The dose the user may effectively set and inject is restricted to a certain value.

On the contrary, the term "variable dose" can be characterized as a dose where the user is substantially free to choose the amount of medicament he wants to inject. The dose is variably adjustable, normally between upper and lower limits.

During the actuation movement, which may correspond to a longitudinal displacement of the actuator in a distal direction parallel to the first and/or the second longitudinal axis, the user may move the actuator towards the distal end of the housing. By moving the setting element from the first into the second position during the setting movement, which is preferably a rotational movement of the setting element with respect to the second longitudinal axis, a preferably fixed dose of a secondary medicament in the secondary drug delivery assembly may be set. The setting movement comes along with the setting element driving the actuation collar into engagement with the actuator.

The setting movement is preferably manually conducted by the user by operating the secondary dose setting mechanism of the secondary drug delivery assembly such as rotating a dose knob via the setting element. Accordingly, the setting element may be rotated relative to a longitudinal axis of the secondary drug delivery assembly from the first position into a second position.

A resetting movement may be characterized as a reversed setting movement, in which the setting element is moved from the second position (e.g. 10 units) back into the first position (e.g. 0 units). Another medicament dose may then set.

Preferably, the setting element is configured such that it moves the actuation collar from the non-engagement position into the engagement position with the actuator only after the setting element has been moved a predetermined distance during the setting movement. Thereby, it can be ensured that the engagement between the actuator and the actuation collar takes place only when the setting element has been moved around the second longitudinal axis about a distance that covers more than zero percent of the entire distance of the setting movement. In other words, the setting movement has to reach a certain distance before the engagement between the actuator and the actuation collar takes place. Preferably, the setting element and the actuation collar are configured such that the predetermined distance corresponds to the distance between the first and the second position of the setting element such that by the setting element reaching the second position during the setting movement, the setting element moves the actuation collar into engagement with the actuator. This advantageously enables for a drug delivery device where a predetermined minimum dose or a complete fixed dose in the second drug delivery assembly has to be set before the actuation collar is coupled to the actuator and the actuation movement induced by the user can be transferred into the second receiving section, respectively to the actuation collar.

The drug delivery assemblies to be received in the receiving sections may be respectively configured to receive a reservoir containing a medicament. Each of the primary reservoir and the secondary reservoir may be a replaceable medicament container such as a cartridge containing a medicament. Actuation of the primary and the secondary dose dispensing mechanism may be triggered by the actuator which is arranged such that it is movable along a first longitudinal axis of the housing. Setting the dose of the secondary medicament with the secondary dose setting mechanism automatically establishes a mechanical connection for actuating the dispense of a dose of a first medicament set with the primary dose setting mechanism and the set second dose by a single actuator.

The actuator and the actuation collar may serve as a linkage component. The actuator in the first receiving section may be arranged such that an actuation movement operates the primary dose dispensing mechanism of the primary drug delivery assembly wherein the actuation collar may be arranged such that it operates the secondary dose dispensing mechanism of the secondary drug delivery assembly when the actuation movement is transferred to the actuation collar. This enables the user to first set a primary dose of medicament in the primary drug delivery assembly and then to set a secondary dose of medicament in the secondary drug delivery assembly. The primary dose dispensing mechanism of the primary drug delivery assembly is not linked to the secondary dose dispensing mechanism when the dose of primary medicament is being set. When the dose of the secondary medicament in the secondary drug delivery assembly is being set, the primary dose dispensing mechanism is linked to the secondary drug dispensing mechanism via the actuator. The setting element may be configured for pure rotational or helical movement with combined translational and rotational movement. Preferably, the actuation collar and the setting element are coaxially arranged, respectively such that they both may rotate about a common axis. Alternatively, the actuation collar and the setting element are arranged having their respective rotation axis. Preferably, the two rotation axes are arranged in parallel to each other.

According to a further embodiment, the setting element is configured to transfer rotational movement onto the actuation collar over at least a sector or part of the setting movement. For example, the entire setting movement may extend over a circular sector of 60°. When the setting element has been rotated over 50°, it only then engages the actuation collar and transfers the setting movement over the remaining 10° to the actuation collar and the actuation collar is caused to engage the actuator. Accordingly, during the setting movement, rotational movement of the setting element is only partly transferred to the actuation collar.

The setting element may be rotationally movable with respect to the actuation collar between a first relative position and a second relative position. The actuation collar may be movable with respect to the setting element in a longitudinal direction. During a first phase of the setting movement, the setting element may rotate with respect to the actuation collar. In a following second phase of the setting movement, an abutment surface provided on the setting element may engage a corresponding counter surface on the actuation collar upon which rotational movement of the setting element is transferred to the actuation collar such that the setting element drives the actuation collar in a rotational motion which leads to the engagement of the first engagement section and the second engagement section. By providing a possible relative movement between the setting element and the actuation collar over at least a part of the circumference of the setting element, the setting process performed by the user is not influenced by the mechanics of the engagement section which provides for a convenient and safe dose setting process of the secondary drug delivery assembly.

The actuator may be rotationally constrained with respect to the housing to ensure a rigid engagement between the first and the second engagement section. The actuator may comprise an actuating surface extending at least partially over the first receiving section in axial direction. The actuator may be configured such that the actuation button is located outside the housing, while the engagement section is inside the housing.

It has been proven effective when the first engagement section and the second engagement section are configured for meshed engagement. The first and the second engagement section may be respectively configured as toothed gear racks. The first and second engagement section may be configured and arranged in the housing such that they are brought into engagement by relative rotation. Preferably, the second engagement section is rotatable with respect to the first engagement section. The teeth may respectively comprise an abutment surface wherein the abutment surfaces may be arranged such that they are aligned perpendicular to the axis of the actuation movement so that the actuation movement is effectively transferred from the first engagement section to the second engagement section.

According to a further aspect of the invention, the first and/or the second engagement section may have a chamfered flank on the backside of the abutment surface. Preferably, the chamfered flank of the first engagement section is arranged such that it substantially faces the proximal end of the housing while the chamfered flank of the second engagement section is directed to the distal end of the housing.

To prevent an improper disengagement of the first and the second receiving section, the first and the second engagement section may be respectively connected to a latching portion. The latching portions may be configured to be driven into latching engagement when the setting element is moved from the first position into the second position. Accordingly, when the setting element drives the actuation collar into engagement with the actuator such that the actuation movement can be transferred to the actuation collar, the latching elements engage. This helps to reduce the likelihood of disengagement of the first and the second engagement section and avoids consequences of improper misuse, i.e. when the patient drops the device. Each of the latching portions on the first and the second engagement section may be configured to extend substantially parallel to the first longitudinal axis. Latching elements may be provided in the latching portions and may be configured to prevent relative rotational movement between the first and the second engagement section. When the first and the second engagement sections are engaged, the latching portions provide for a resisting force such that the first and the second engagement section are rotationally constrained with respect to each other.

According to a further embodiment of the invention, the actuation collar comprises at least one securing means such as a protruding rib, preferably extending parallel to a commonly shared rotation axis of the setting element and the actuation collar. The protruding rib may be configured to engage at least one groove formed on the actuator and extending parallel to the first longitudinal axis. The arrangement of the groove and the rib may be opposite as well with the rib being provided on the actuator and the groove being provided on the actuation collar. The at least one protruding rib may also be provided on an inner surface of the housing. It is preferably configured such as to secure a latched position of the actuation collar relative to the housing when the rib and the groove are engaged. The rib and groove connection may be provided for latching engagement and be located on the latching portions. The latching portions may be configured such that they engage each other in a snap-like manner. Such snap connection is not only beneficial in terms of securing the engagement between the first and the second engagement section but may be used to give the user a sensible feedback indicating that the mechanical link between the first and the second engagement section is established.

The housing may further comprise a locking element configured to rigidly engage the first and/or the second drug delivery assembly and to be detachably received within the housing. The housing may comprise at least two housing parts configured to rigidly receive the locking element when the housing parts are assembled. The locking element may be configured to be attached to drug delivery assemblies that have already passed approval procedures before the competent authorities. The locking element may be configured to rigidly engage the first and/or the second drug delivery assembly so that the first and/or the second drug delivery assembly is/are rotationally and/or longitudinally constrained with respect to the housing when received in the respective receiving section. The locking element may be configured as a coupling element, a locking or coupling collar, a spacer or an insert.

The drug delivery assemblies may comprise at least two housing parts wherein the locking element is adapted to be attached to attachment means provided on the drug delivery assembly housing parts for mutual attachment of the two housing parts. In other words, the locking element may be adapted to be fixed to attachment means on the drug delivery assembly wherein those attachment means are normally used to assemble the single parts of the drug delivery assembly. Here, the locking element may serve as a spacer, for example. Furthermore, with the locking element attached, the relative position between the primary drug delivery assembly and the secondary drug delivery assembly can be efficiently secured. Accordingly, the locking element may also serve as coupling element.

According to a further embodiment of the invention, a receiving element may be provided, which is configured to be attached to a primary dose setting mechanism of the first drug delivery assembly. Preferably, the receiving element is arranged rotatably with respect to the housing. The receiving element may be configured as a user-operable dose setter. The receiving element may be a sleeve-like dial element configured to accommodate at least a portion or element of the primary dose setting mechanism such as a dose dial sleeve or a dose dial knob or a dose setter so that rotational movement of the receiving element is directly transferred to the primary dose setting mechanism. According to a further embodiment of the invention, the receiving element may be configured to receive the primary dose dispensing mechanism.

The actuator may be movable relative to the receiving element in the axial direction respectively in the direction of the actuation movement of the actuator from a first preferably proximal position to a second preferably distal position during the actuation movement. If a primary drug delivery assembly is received in the housing, the actuator may be arranged to actuate the primary dose dispensing mechanism when being set into the second position.

A locking unit switchable resp. movable between a locked and an unlocked position may be arranged such that in the locked position movement of the actuator from the first into the second position is prevented and in the unlocked position movement of the actuator from the first into the second position is allowed. The locking unit may be coupled to the setting element such that the setting movement sets the locking unit from the locked into the unlocked position. Accordingly, the switching of the locking unit is coupled to the setting of the secondary drug delivery assembly such that actuation of the primary drug delivery assembly without setting a dose in the secondary drug delivery assembly is prevented.

The present invention also concerns a drug delivery device which comprises a housing as described herein, wherein the first receiving section retains a primary drug delivery assembly with a primary dose setting mechanism to set a dose of a primary medicament, which may be received in a primary reservoir, and a primary dose dispensing mechanism to dispense a set dose of the primary medicament through a dispense interface, wherein the primary dose dispensing mechanism is configured to be actuated by the actuator during the actuation movement of the actuator. In the second receiving section, a second drug delivery assembly is retained, which comprises a secondary dose setting mechanism to set a dose of a secondary medicament, which may be received in a secondary reservoir, and a secondary dose dispensing mechanism to dispense a set dose of the secondary medicament through the dispense interface. The setting element is operably coupled to the secondary dose setting element such that the setting movement of the setting element leads to adjustment or setting of the set dose of the secondary medicament and to the coupling of the actuation movement to the secondary dose dispense mechanism, respectively such that the actuation movement may be transferred to the secondary dose dispense mechanism. Preferably, the size of the dose of the secondary medicament that is set with the setting movement is defined by the length of the setting movement. According to further embodiment, the length of the setting movement is predefined, resp. limited such that the set dose of the secondary medicament is a fixed resp. non-variable dose. Reaching the final value of the predetermined dose of the secondary medicament with the setting movement may result in the coupling of the actuator to the secondary dose dispense mechanism.

The user may individually set a dose of the primary medicament and a dose of the secondary medicament independently before actuating the actuator by directly manipulating the respective dose setting mechanism. For example, the patient may set the dose of the primary medicament and may then set the dose of the secondary medicament. After the secondary dose setting mechanism is operated by the setting element in a setting movement, the mechanical link between the actuator and the actuation collar is established and the actuation movement can be transferred to the secondary dose dispense mechanism. Accordingly, after having set the dose of the primary medicament and the dose of the secondary medicament, the user may operate the actuator. The actuation movement is transferred to the secondary dose dispensing mechanism via the first and the second engagement section and the actuation movement actuates the primary and secondary dose dispensing mechanism.

The actuation collar may at least partially surround the secondary drug delivery assembly in a sleeve-like manner, such that the actuation collar is moved from a first position to a second position relative to a longitudinal axis of the secondary drug delivery assembly wherein the movement from the first position into the second position leads to an engagement with a trigger button or the like of the secondary dose dispensing mechanism such that the secondary dose dispensing mechanism is actuated.

The primary dose setting mechanism and the secondary dose setting mechanism may be a variable dose setting mechanism respectively.

According to a further embodiment of the invention, the secondary drug delivery assembly may comprise a prestressable biasing member such as a spring configured to relax and to dispense the set dose of the secondary medicament when the secondary dose dispensing mechanism is actuated. The biasing member may be configured to be stressed in a dose setting movement and to be relaxed during dose dispense, such that by relaxation of the biasing member the set dose of the secondary medicament is dispensed. Preferably, the dose setting mechanism is reset by the relaxation of the biasing member.

A dose setter such as a dose knob may be rotatably coupled to a secondary assembly housing such that rotation of the knob relative to the secondary assembly housing in a first direction results in prestressing or twisting of the biasing member. The secondary dose dispensing mechanism may include a trigger button, a release button, dispense button or the like provided for releasing the prestressed spring. By releasing the spring, the spring drives a drive member of the dose dispensing mechanism in axial, respectively distal direction relative to the housing of the secondary drug delivery assembly. The secondary drug delivery assembly may further be configured such that by release of a spring force, the dose setting mechanism is rotationally reset in a resetting movement, in which the setting movement of the setting element is reversed and the setting element is moved back from the second position into the first position. Such devices are also known as power assisted injectors, which can be characterized by an energy source such as stored mechanical energy provided by a prestressable spring to drive a medicament out of a cartridge.

Displacement of the drive member may force a bung or the like in the secondary reservoir in distal direction such that secondary medicament is driven out of the secondary reservoir.

In order to provide for a limited fixed dose setting mechanism, the setting element may be provided with rotational abutment surfaces configured to limit a settable dose of the secondary medicament to a maximum settable dose and/or a fixed dose. In other words, the abutment surfaces may limit the setting movement, thereby rendering the settable dose of the secondary medicament to a fixed dose. This may be achieved by providing respective counter abutment surfaces on the housing and/or the secondary drug delivery assembly such as a projection. By limiting the rotational movement of the setting element relative to the secondary drug delivery assembly and/or the housing, the secondary drug delivery assembly may be of the variable dose type, wherein the variable dose is limited to an upper value. Accordingly, the size of the fixed dose is predetermined by the design of the mechanism.

As explained above, it has been proven effective when the setting element is configured such that is drives the actuation collar into engagement with the actuator when the setting element reaches the second position during the setting movement. Thereby, it is ensured, that an actuation movement is only then transferred to the secondary dispensing mechanism when a predetermined dose is completely set. When the second position is not completely reached, the actuation collar is not linked to the actuator and the secondary dispensing mechanism cannot be actuated. Accordingly, the second position of the setting element may correspond to such predetermined dose. With these measures, a minimum settable dose may be defined. By further limiting the rotational movement of the setting element relative to the secondary drug delivery assembly and/or the housing which corresponds to a maximum settable dose as exemplarly explained above, a predetermined fixed dose can be defined. The setting of a dose higher than the fixed dose is prevented, while the setting of a dose lower than the fixed dose results in the actuation collar not engaging the actuator. Then, the set dose in the secondary drug delivery assembly cannot be injected. Hence, the user has to dial a predetermined fixed dose.

A variable dose setting mechanism of the secondary drug delivery assembly can be efficiently turned into a fixed dose setting mechanism. The respective features for determining the fixed dose can be provided by the housing elements or by the housing elements in combination with features provided by the secondary drug delivery assembly. For example, the secondary drug delivery assembly may be provided with a projection or the like on its outer surface and the setting element may be configured to engage the projection when reaching the second position, wherein the engagement between the projection and the setting element prevents further rotation between the setting element and the secondary drug delivery assembly.

The actuator may be provided with a pressing face configured to engage a pressure receiving section of the primary dose dispensing mechanism such as a dose dispense button. The actuator may be arranged such that by the actuation movement of the actuator, a gap between the pressing face and the pressure receiving section is closed and the actuation movement of the actuator is transferred to the primary dose dispense mechanism. Thereby, sequential delivery of the secondary medicament and the primary dose of medicament may be achieved. Not before the gap is closed, the primary dose of medicament is dispensed. However, the actuation movement of the actuator is transferred to the secondary dose dispensing mechanism of the secondary drug delivery assembly before the gap is closed. As a result, the secondary medicament is injected before the primary medicament. This effectively provides for the sequential delivery of two medicaments in one injection step. Accordingly, the gap has a delay function regarding the activation of the primary dose dispense mechanism.

Preferably, the drug delivery device comprises at least one cartridge containing a medicament.

The term "medicament", as used herein, means a pharmaceutical formulation containing at least one pharmaceutically active compound,
wherein in one embodiment the pharmaceutically active compound has a molecular weight up to 1500 Da and/or is a peptide, a proteine, a polysaccharide, a vaccine, a DNA, a RNA, an enzyme, an antibody or a fragment thereof, a hormone or an oligonucleotide, or a mixture of the above-mentioned pharmaceutically active compound,
wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutically active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exendin-3 or exendin-4 or an analogue or derivative of exendin-3 or exendin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyheptadecanoyl) human insulin.

Exendin-4 for example means Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
H-(Lys)5-des Pro36, des Pro37 Exendin-4(1-39)-NH2,
des Pro36 Exendin-4(1-39),
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(O2)25, Asp28] Exendin-4(1-39),
des Pro36 [Trp(O2)25, IsoAsp28] Exendin-4(1-39),
des Pro36 [Met(O)14 Trp(O2)25, Asp28] Exendin-4(1-39),
des Pro36 [Met(O)14 Trp(O2)25, IsoAsp28] Exendin-4(1-39); or
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(O2)25, Asp28] Exendin-4(1-39),
des Pro36 [Trp(O2)25, IsoAsp28] Exendin-4(1-39),
des Pro36 [Met(O)14 Trp(O2)25, Asp28] Exendin-4(1-39),
des Pro36 [Met(O)14 Trp(O2)25, IsoAsp28] Exendin-4(1-39),
wherein the group -Lys6-NH2 may be bound to the C-terminus of the Exendin-4 derivative;
or an Exendin-4 derivative of the sequence
des Pro36 Exendin-4(1-39)-Lys6-NH2 (AVE0010),
H-(Lys)6-des Pro36 [Asp28] Exendin-4(1-39)-Lys6-NH2,
des Asp28 Pro36, Pro37, Pro38Exendin-4(1-39)-NH2,
H-(Lys)6-des Pro36, Pro38 [Asp28] Exendin-4(1-39)-NH2,
H-Asn-(Glu)5des Pro36, Pro37, Pro38 [Asp28] Exendin-4(1-39)-NH2,
des Pro36, Pro37, Pro38 [Asp28] Exendin-4(1-39)-(Lys)6-NH2,
H-(Lys)6-des Pro36, Pro37, Pro38 [Asp28] Exendin-4(1-39)-(Lys)6-NH2,
H-Asn-(Glu)5-des Pro36, Pro37, Pro38 [Asp28] Exendin-4(1-39)-(Lys)6-NH2,
H-(Lys)6-des Pro36 [Trp(O2)25, Asp28] Exendin-4(1-39)-Lys6-NH2,
H-des Asp28 Pro36, Pro37, Pro38 [Trp(O2)25] Exendin-4(1-39)-NH2,
H-(Lys)6-des Pro36, Pro37, Pro38 [Trp(O2)25, Asp28] Exendin-4(1-39)-NH2,
H-Asn-(Glu)5-des Pro36, Pro37, Pro38 [Trp(O2)25, Asp28] Exendin-4(1-39)-NH2,
des Pro36, Pro37, Pro38 [Trp(O2)25, Asp28] Exendin-4(1-39)-(Lys)6-NH2,
H-(Lys)6-des Pro36, Pro37, Pro38 [Trp(O2)25, Asp28] Exendin-4(1-39)-(Lys)6-NH2,
H-Asn-(Glu)5-des Pro36, Pro37, Pro38 [Trp(O2)25, Asp28] Exendin-4(1-39)-(Lys)6-NH2,
H-(Lys)6-des Pro36 [Met(O)14, Asp28] Exendin-4(1-39)-Lys6-NH2,
des Met(O)14 Asp28 Pro36, Pro37, Pro38 Exendin-4(1-39)-NH2,
H-(Lys)6-desPro36, Pro37, Pro38 [Met(O)14, Asp28] Exendin-4(1-39)-NH2,
H-Asn-(Glu)5-des Pro36, Pro37, Pro38 [Met(O)14, Asp28] Exendin-4(1-39)-NH2,
des Pro36, Pro37, Pro38 [Met(O)14, Asp28] Exendin-4(1-39)-(Lys)6-NH2,
H-(Lys)6-des Pro36, Pro37, Pro38 [Met(O)14, Asp28] Exendin-4(1-39)-(Lys)6-NH2,
H-Asn-(Glu)5 des Pro36, Pro37, Pro38 [Met(O)14, Asp28] Exendin-4(1-39)-(Lys)6-NH2,
H-Lys6-des Pro36 [Met(O)14, Trp(O2)25, Asp28] Exendin-4(1-39)-Lys6-NH2,
H-des Asp28 Pro36, Pro37, Pro38 [Met(O)14, Trp(O2)25] Exendin-4(1-39)-NH2,
H-(Lys)6-des Pro36, Pro37, Pro38 [Met(O)14, Asp28] Exendin-4(1-39)-NH2,
H-Asn-(Glu)5-des Pro36, Pro37, Pro38 [Met(O)14, Trp(O2)25, Asp28] Exendin-4(1-39)-NH2,
des Pro36, Pro37, Pro38 [Met(O)14, Trp(O2)25, Asp28] Exendin-4(1-39)-(Lys)6-NH2,
H-(Lys)6-des Pro36, Pro37, Pro38 [Met(O)14, Trp(O2)25, Asp28] Exendin-4(S1-39)-(Lys)6-NH2,
H-Asn-(Glu)5-des Pro36, Pro37, Pro38 [Met(O)14, Trp(O2)25, Asp28] Exendin-4(1-39)-(Lys)6-NH2;
or a pharmaceutically acceptable salt or solvate of any one of the afore-mentioned Exendin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Antibodies are globular plasma proteins (-150 kDa) that are also known as immunoglobulins which share a basic structure. As they have sugar chains added to amino acid residues, they are glycoproteins. The basic functional unit of each antibody is an immunoglobulin (Ig) monomer (containing only one Ig unit); secreted antibodies can also be dimeric with two Ig units as with IgA, tetrameric with four Ig units like teleost fish IgM, or pentameric with five Ig units, like mammalian IgM.

The Ig monomer is a "Y"-shaped molecule that consists of four polypeptide chains; two identical heavy chains and two identical light chains connected by disulfide bonds between cysteine residues. Each heavy chain is about 440 amino acids long; each light chain is about 220 amino acids long. Heavy and light chains each contain intrachain disulfide bonds which stabilize their folding. Each chain is composed of structural domains called Ig domains. These domains contain about 70-110 amino acids and are classified into different categories (for example, variable or V, and constant or C) according to their size and function. They have a characteristic immunoglobulin fold in which two β sheets create a "sandwich" shape, held together by interactions between conserved cysteines and other charged amino acids.

There are five types of mammalian Ig heavy chain denoted by α, δ, ε, γ, and µ. The type of heavy chain present defines the isotype of antibody; these chains are found in IgA, IgD, IgE, IgG, and IgM antibodies, respectively.

Distinct heavy chains differ in size and composition; α and γ contain approximately 450 amino acids and δ approximately 500 amino acids, while µ and ε have approximately 550 amino acids. Each heavy chain has two regions, the constant region (C_{H}) and the variable region (V_{H}). In one species, the constant region is essentially identical in all antibodies of the same isotype, but differs in antibodies of different isotypes. Heavy chains γ, α and δ have a constant region composed of three tandem Ig domains, and a hinge region for added flexibility; heavy chains µ and ε have a constant region composed of four immunoglobulin domains. The variable region of the heavy chain differs in antibodies produced by different B cells, but is the same for all antibodies produced by a single B cell or B cell clone. The variable region of each heavy chain is approximately 110 amino acids long and is composed of a single Ig domain.

In mammals, there are two types of immunoglobulin light chain denoted by λ and κ. A light chain has two successive domains: one constant domain (CL) and one variable domain (VL). The approximate length of a light chain is 211 to 217 amino acids. Each antibody contains two light chains that are always identical; only one type of light chain, κ or λ, is present per antibody in mammals.

Although the general structure of all antibodies is very similar, the unique property of a given antibody is determined by the variable (V) regions, as detailed above. More specifically, variable loops, three each the light (VL) and three on the heavy (VH) chain, are responsible for binding to the antigen, i.e. for its antigen specificity. These loops are referred to as the Complementarity Determining Regions (CDRs). Because CDRs from both VH and VL domains contribute to the antigen-binding site, it is the combination of the heavy and the light chains, and not either alone, that determines the final antigen specificity.

An "antibody fragment" contains at least one antigen binding fragment as defined above, and exhibits essentially the same function and specificity as the complete antibody of which the fragment is derived from. Limited proteolytic digestion with papain cleaves the Ig prototype into three fragments. Two identical amino terminal fragments, each containing one entire L chain and about half an H chain, are the antigen binding fragments (Fab). The third fragment, similar in size but containing the carboxyl terminal half of both heavy chains with their interchain disulfide bond, is the crystalizable fragment (Fc). The Fc contains carbohydrates, complement-binding, and FcR-binding sites. Limited pepsin digestion yields a single F(ab')2 fragment containing both Fab pieces and the hinge region, including the H-H interchain disulfide bond. F(ab')2 is divalent for antigen binding. The disulfide bond of F(ab')2 may be cleaved in order to obtain Fab'. Moreover, the variable regions of the heavy and light chains can be fused together to form a single chain variable fragment (scFv).

Pharmaceutically acceptable salts are for example acid addition salts and basic salts. Acid addition salts are e.g. HCl or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

In the following, the invention will be described by way of examples and with reference to the schematic drawings in which:
- Fig. 1: shows in a perspective exploded view the inventive drug delivery device;
- Fig. 2a to 2c: show a single dispense interface hub and the attachment to the housing of Fig. 1;
- Fig. 3: shows in perspective view a portion of the drug delivery device of Fig. 1 with a part of the housing removed;
- Figs. 4a to 4d: show parts of the drug delivery of Fig. 1 during a dose setting and dose dispensing sequence;
- Figs. 5a, 5b: show a setting sequence of the secondary dose setting mechanism;
- Figs. 6: shows a cross-sectional view of a detail of the drug delivery device;
- Figs. 7a to 7c: show in a perspective view a further detail of the drug delivery device; and
- Figs. 8a to 8b: show in a perspective view parts of a drug delivery device in accordance with a second embodiment of the present invention.

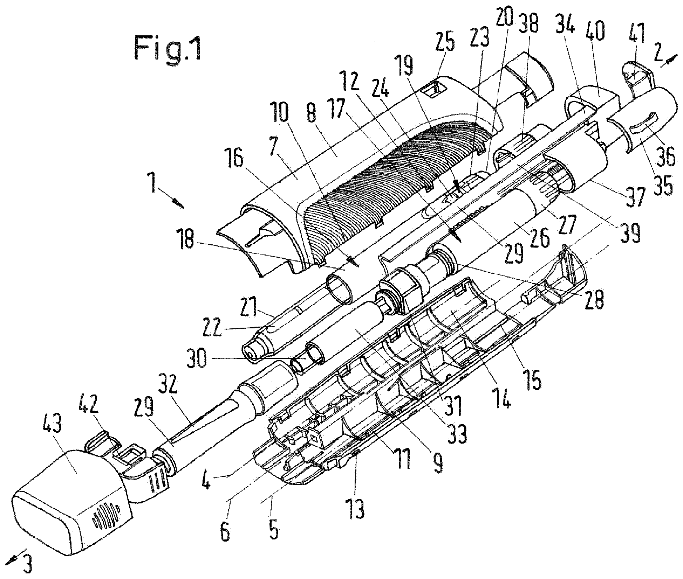

Figure 1 shows of a drug delivery device 1 with a proximal end 2 and a distal end 3. A first longitudinal axis 4 and a second longitudinal axis 5 run in a parallel relationship parallel to a longitudinal axis 6 of a housing 7. The housing 7 comprises a first upper half shell 8 and a second lower half shell 9.

The first half shell 8 and the second half shell 9 are connectable at side edges 10, 11. When the drug delivery device 1 is assembled, first side edges 10 of the first half shell 8 engage second side edges 11 of the second half shell 9 and the first and the second half shell 8, 9 accommodate the majority of components disposed between the first half shell 8 and the second half shell 9 in Figure 1 as explained further below.

The first side edges 10 and the second side edges 11 are provided with corresponding snap features 12 and 13 which ensure a fixed installation of the first and second half shell 8, 9. In an exemplary embodiment, each of the half shells 8, 9 is provided with eight snap features 12, 13 at their side edges arranged such as to engage its respective counterpart on the other of the half shells 8, 9.

A first receiving section 14 of the housing 7 extends from the proximal end 2 to the distal end 3 of the housing 7 and is disposed substantially parallel to a second receiving section 15 of the housing 7. The first longitudinal axis 4 runs through the first receiving section 14 while the second longitudinal axis 5 runs through the second receiving section 15.

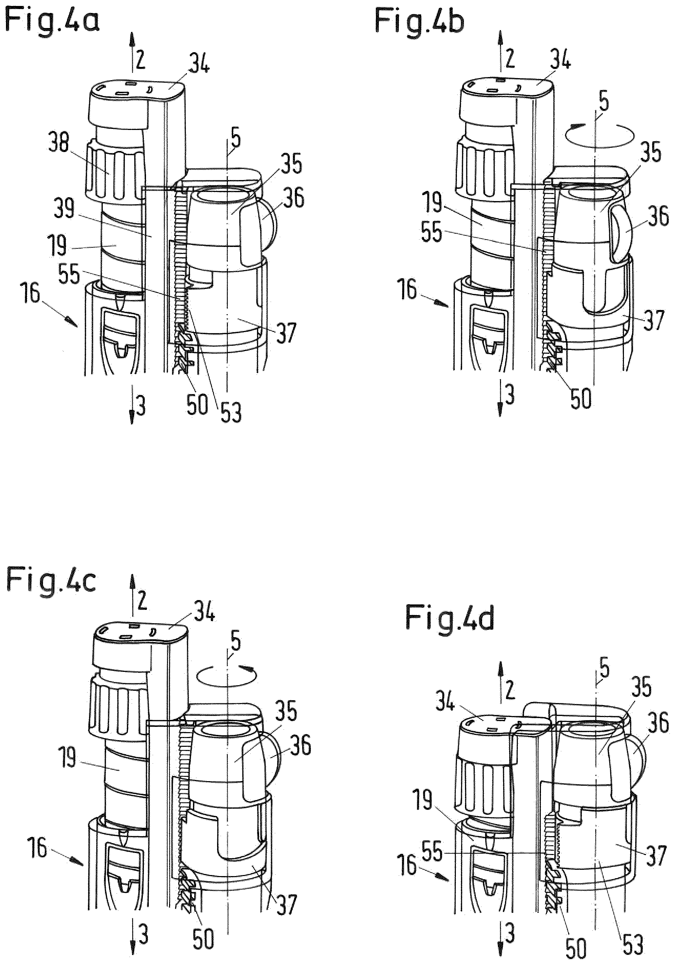

If the drug delivery device 1 is assembled, the first receiving section 14 retains a primary drug delivery assembly 16 and the second receiving section 15 retains a secondary drug delivery assembly 17. The primary drug delivery assembly 16 is a pen-type injector of the kind that provides for administration by injection of medicinal products from a multi dose cartridge. The primary drug delivery assembly 16 comprises a primary assembly housing 18 with a piston rod (not shown) having a screw thread, an insert which is located in the primary assembly housing 18 and through which the piston rod may rotate. The insert is secured to the housing 18 and the piston rod is threadedly engaged with an inner thread provided on the insert. A drive sleeve (not shown) is rotatable with respect to the piston rod during dose setting (including dose cancelling) and is axially displaceable but not rotatable with respect to the piston rod during dose delivery. A dose dial sleeve 19 (see Figure 4a) is accommodated in and rotatable with respect to the primary assembly housing 18. The dose dial sleeve 19 engages the primary assembly housing 18 with an outer screw thread which engages an inner screw thread on the primary assembly housing 18 or a threaded housing insert such that rotation of the dose dial sleeve 19 relative to the primary assembly housing 18 leads to axial displacement of the dose dial sleeve 19 relative to the primary assembly housing 18. The dose dial sleeve 19 is provided with a series of numbers on its outer surface which are visible through an opening or window of housing 18.

A primary dispense button 20 is located on the drive sleeve at its proximal end. The primary assembly housing 18 contains a clutch means (not shown) which upon depression of the primary dispense button 20 allows relative rotation between the dose dial sleeve 19 and the drive sleeve. A spring (not shown) is provided biasing the clutch and the primary dispense button 20 into a position releasably coupling the dial sleeve 19 and the drive sleeve such that they are rotationally constrained. The piston rod and an internal surface of the drive sleeve are in a threaded engagement such that the drive sleeve drives the piston rod upon longitudinal displacement of the drive sleeve during dose dispensing.

The piston rod extends in axial direction of the primary assembly housing 18 and is provided with a pressure foot at its distal end. At the distal end of the primary assembly housing 18, a cartridge holder 21 is releasable attached to the primary assembly housing 18. The cartridge holder 21 receives a primary cartridge 22 (primary container) containing a primary medicament. The distal end of the primary cartridge 22 is closed with a septum.

For setting a dose, the operator must first select a dose. To set a dose, the dose dial sleeve 19 is rotated by manipulating a distal end section configured as a dose setter 23. The dose setter 23 is rotated until the desired dose value is visible through a first window 24 in the primary assembly housing 18 and a second window 25 or opening in the first half shell 8. When the drug delivery device 1 is assembled, the first window 24 and the second window 25 coincide. The rotation of the dose setter 23 moves the dose dial sleeve 19 in proximal direction with respect to the housing 18 in a helical movement.

The drive sleeve rotates together with the dose dial sleeve 19 relative to the piston rod. The piston rod does not rotate during dose setting. The drive sleeve is carried in proximal direction along the piston rod by the dose dial sleeve as the dose dials sleeve 19 moves out of the primary assembly housing 18.

Once the desired dose has been set, in order to deliver the dose, the primary dispense button 20 is moved in distal direction. When the primary dispense button 20 is depressed, clutch means provided between the dose dial sleeve 19 and the drive sleeve allow relative rotation between these components and rotationally constrain the drive sleeve to housing 18 so that longitudinal displacement of the primary dispense button 20 in the distal direction causes the drive sleeve to move axially towards the distal end of the primary drug delivery assembly 16, while the dose dial sleeve 19 rotates back into the housing 18. Since the piston rod is threaded to the drive sleeve, the piston rod is rotated through the insert in distal direction, thereby advancing a piston in the primary cartridge 22 such that the set dose of the primary medicament is dispensed through a dispense interface fluidly connected to the primary cartridge 22 at its distal end.

The secondary drug delivery assembly 17 is also a pen-type injector with a user operable dose setting mechanism. The secondary drug delivery assembly 17 is known in the art as being of the power assisted injector type and comprises a prestressable biasing member (not shown) configured to relax and to automatically dispense a set dose of a secondary medicament when a secondary dose dispensing mechanism is actuated. A secondary assembly housing 26 accommodates the secondary dose setting mechanism, which comprises a dose knob 27 rotatably coupled to the secondary assembly housing 26 at its proximal end. An outer screw thread 28 is provided at the distal end of a secondary assembly housing 26. A cartridge holder 29 is located at the distal end of the secondary drug delivery assembly 17 and receives a disposable secondary cartridge 30 (secondary container) containing a secondary medicament. The distal end of the secondary cartridge 30 is closed with a septum while a bung (not shown) seals the proximal end of the cartridge.

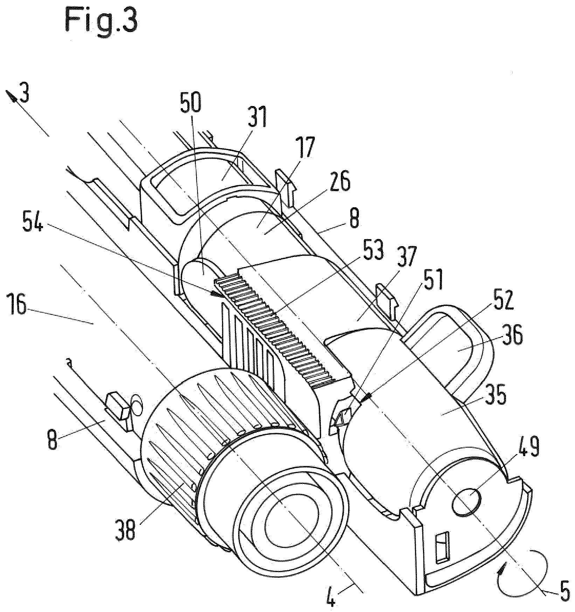

In the secondary assembly housing 26 there is arranged the secondary dose setting mechanism and a secondary dose dispensing mechanism. The secondary dose dispensing mechanism includes a trigger button (reference numeral 50 in Figure 3) that is movable from a proximal to a distal position relative to the secondary assembly housing 26. When the trigger button is moved into the distal position, the secondary dose dispensing mechanism is actuated.

A biasing element such as a torsion spring (not shown) is provided within the secondary assembly housing 26 and is coupled to a drive member (not shown). The dose setting knob 27 is coupled to the torsion spring such that during a setting movement to set a dose of the secondary medicament for injection, by rotating the dose knob 27 relative to the secondary assembly housing 26 in a first direction, the spring is prestressed. Actuation of the trigger button releases the spring. By releasing the spring, the spring forces the drive member to move through the secondary assembly housing 26 in distal direction. The displacement of the drive member is proportional to the aforesaid dose, which corresponds to the degree of rotation of the dose knob 27 relative to the secondary assembly housing 26.

Between the cartridge holder 29 and the secondary assembly housing 26 a locking element configured as a collar 31 is provided. As in the depicted embodiment the second drug delivery assembly 17 is of the power assisted injector type, the collar 31 is referred to as "auto collar" 31. The auto collar 31 has an internal screw thread provided at its proximal end for attachment to the external thread 28 at the distal end of the secondary assembly housing 26 and an external thread provided at its distal end for attachment to the cartridge holder 29 which is provided with a corresponding internal thread at its proximal end. The setup of the internal screw thread of the auto collar 31 corresponds to the internal thread of the cartridge holder 29, while the external thread of the auto collar 31 corresponds to the external thread of the secondary assembly housing 26. Thereby, the auto collar 31 constitutes an inserted intermediate piece that lengthens the overall length of the secondary drug delivery assembly 17 in axial direction.

The secondary drug delivery assembly 17 is usually produced and delivered by the manufacturer with the internal screw thread of the cartridge holder 29 directly engaging the outer screw thread 28 of the secondary housing assembly 26, hence, without the auto collar 31. In the depicted embodiment, the auto collar 31 is interposed therebetween to lengthen the overall length of the secondary drug delivery assembly 17, i.e. to adapt it to the length of the primary drug delivery assembly 16. It goes without saying that the provision of the auto collar may be omitted or that a similar means may be provided for the primary drug delivery assembly if required. In addition, the dimensions of the housing or its components may be adapted to fit various sizes of the primary and secondary drug delivery assemblies.

The cartridge holder 29 of the secondary drug delivery assembly 17 is provided with a window 32 through which a cartridge plunger 33 is visible which has been added to the secondary drug delivery assembly 17 as the auto collar 31 has. The cartridge plunger 33 is axially movable received within the cartridge holder 29. The cartridge plunger 33 is a sleeve-like element having a proximal end and may be driven in distal direction by the drive member of the secondary dose dispensing mechanism (for example a piston rod) wherein the drive member extends through an inner opening extending through the auto collar 31 to push against the proximal end of the cartridge plunger. The distal end of the cartridge plunger 33 has a distal opening which receives a proximal section of the secondary cartridge 30. The proximal end of the opening is provided with a pressure foot which abuts the bung of the secondary cartridge 30 when the cartridge plunger 33 moves distally with respect to the secondary cartridge 30. The cartridge plunger 33 forces the bung in distal direction when driven by the drive member such that the secondary medicament from the secondary cartridge 30 is expelled through the dispense interface attached to the distal end of the housing.

In the depicted embodiment, the original cartridge (not shown) of the power assisted-injector 17 has been replaced by the secondary cartridge 30 which is regarding its outer-diameter and inner volume smaller than the original cartridge. When the cartridge plunger 33 is displaced in distal direction by the drive member of the secondary dose mechanism, the cartridge plunger 33 starts to cover the outer surface of the secondary cartridge 30 and a dose of the secondary medicament is forced out of the secondary cartridge. Because the secondary cartridge 30 is smaller than the original cartridge the herein shown power assisted injector has been layed out such that a dose that is set with the secondary dose setting mechanism is larger than the dose that is actually dispensed upon actuation. In other words, the size of the secondary cartridge 30 has been chosen with regard to its inner diameter that the amount of secondary medicament that is expelled during dispensing the dose is half the dose that set with the dose knob 27.

The outer surface of the cartridge plunger 33 is provided with other visual indicators. As the displacement of the cartridge plunger 33 relative to the secondary cartridge 30 corresponds to the amount of secondary medicament being expelled from the secondary cartridge 30, the position of the cartridge plunger 33 relative to the secondary cartridge 30 indicates the patient the amount of secondary medicament that is left in the secondary cartridge 30. It is possible to provide a marking on the outer surface of the cartridge plunger, e.g. with a sticker, a label or the like. The marking gives the patient a visual indication of the decreasing filling level over a period of his treatment, e.g. through an opening or window in the housing. For example, a number of rings provided on the outer circumference of the cartridge plunger that may be arranged one behind each other in axial direction may indicate resp. present to the number of days, the patient has already used the device for his treatment. With each injection, the plunger moves in distal direction over a distance corresponding to the volume of medicament that is dispensed and an increasing number of rings becomes visible by moving into the field of vision though the window or opening in the housing. By a change of color in the rings, e.g. from blue to red, when the amount of medicament that is left in the cartridge reaches a minimum level, the patient is reliably informed about the upcoming depletion of the medicament.

The housing 7 further comprises an actuator 34 and setting element 35 with a lever 36 to enable the user to conveniently set a dose with the secondary medicament, an actuation collar 37 and a receiving element 38 for the dose setter 23 configured to be manipulated by the patient to set a dose of the primary medicament. The actuator 34 comprises an elongated bar 39 extending parallel to the first longitudinal axis 4 and an actuation section 40 which extends substantially perpendicular to the bar 39. The actuator 34 is mainly located in the first receiving section 14 and the bar 39 substantially extends between the primary drug delivery assembly 16 and the secondary drug delivery assembly 17. The actuator is slidably guided in the housing 7 by respective formed guidance section, which may include webs or the like (not shown). The receiving element 38 is located between the primary dispense button 20 and the actuation section 40. The actuation section 40 is located at the proximal end of the actuator 34. A button cap 41 is coupled to the proximal end of the actuation section 40.

A single dispense interface 42 is releasably attachable to the distal end 3 of the housing 7. The single dispense interface 42 is a needle hub having two proximal needles to pierce the septum of each of the primary cartridge 22 and the secondary cartridge 30, wherein the two proximal needles are fluidly connected to an attachment section 47 located at the distal end of the single dispense interface 42. An injection needle (not shown) is attachable to the attachment section 47 for injecting the primary medicament and the secondary medicament into the skin of a patient. A cap 43 is releasably attachable to the distal end of the drug delivery 1 to cover the distal end 3 of the drug delivery device 1 and the single dispense interface 42 when the device is not in use. Of course, the single dispense interface 42 could be configured as non-detachable, wherein the single dispense interface 42 is not removable from the housing 7 after having been attached. The drug delivery device 1 may then be provided as a disposable injection device. Such devices can be thrown away or recycled after the content of one or both of the medicaments has been exhausted.

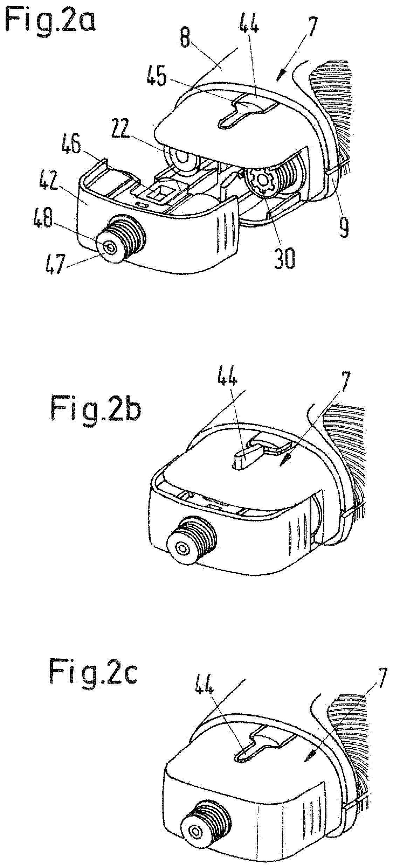

In Figures 2a to 2c, the drug delivery device is from Figure 1 is assembled together with the first half shell 8 and a second half shell 9 being attached to each other. The distal end of the primary cartridge 22 and the distal end of the secondary cartridge 30 are accessible. For attachment, the housing 7 and the single dispense interface 42 are provided with releasable locking features comprising a flexible snap feature 44 with an external bump 45. On the inside of the housing 7, the snap feature 44 has a projection (not shown) configured to engage a recess 46 provided on the dispense interface 42. As can be seen from Figures 2a to 2c, the proximal end of the dispense interface 42 is inserted into the distal end of the housing 7. During attachment, the section of the dispense interface 42 lying proximally from the recess 46 lifts resp. tilts the snap feature 44 as shown in Figure 2b. As a result, the snap feature 44 visibly extends from the upper surface of the housing 7 indicating that the dispense interface is not yet sufficiently inserted into the housing 7. The snap feature 44 gives visually impaired patients a reliable tactile feedback regarding a proper attachment process, in particular.

When the dispense interface 42 is properly attached to the housing 7, the two proximal needles (not shown) of the dispense interface 42 pierce the septa of the primary cartridge 22 and the secondary cartridge 30. At the distal end of the dispense interface 42, the attachment section 47 for attachment of the injection needle is provided. The attachment section 47 is configured as an outer thread with a central opening 48. The central opening 48 is configured to receive a proximal end of an injection needle wherein attachment of the injection needle establishes fluid communication with both of the proximal needles of the single dispense interface. With the distal end of the injection needle the primary medicament and the secondary medicament are injected through the skin of a patient. When the dispense interface 42 is properly attached to the drug delivery device 1 and when the distal injection needle is attached to the attachment section 47, fluid connection between the primary cartridge 22, the secondary cartridge 30 and the injection needle is established.

The dispense interface 42 is properly attached to the housing 7 when the 44 snap feature snaps into the recess 46. Then, the outer surface at distal end of the housing 7 is flat and the outer surface of the snap feature 44 (with the exception of the external bump 45) lies substantially flush with the outer surface of the housing 7 which indicates that the dispense interface 42 is properly attached to the drug delivery device.

The bump feature 45 is provided to engage a corresponding protrusion or recess (not shown) in the inner surface of the cap 43 when the cap 43 is attached to the distal end of the housing 7 (see figure 1), thereby ensuring the tight fit of the cap 43 on the housing 7.

Figure 3 shows the primary drug delivery assembly 16 and the secondary drug delivery assembly 17 received in the first half shell 8. The setting element 35 is attached to the dose knob of the secondary drug delivery assembly 17 such that rotation of the setting element 35 about the second longitudinal axis 5 is directly transferred to the dose knob. A proximal end of the setting element 35 is received within a closed ring 49 provided by the first half shell 8 to ensure that the axis of rotation of the setting element 35 remains properly aligned. This ensures that the setting element 35 does not tilt before, during and after rotation of the setting element 35 around the second longitudinal axis 5. The auto collar 31 rigidly engages the secondary drug delivery assembly 17 and is also detachably received within the housing, wherein inner surfaces of first half shell 8 and the second half shell comprise a number of webs which rigidly accommodate the actuation collar 31 when the housing parts 8, 9 are assembled. Thereby, the secondary drug delivery assembly 17 is rotationally and axially constrained by the auto collar 31 with respect to the housing 7. This ensures the correct position of the secondary drug delivery assembly 17 within the housing and relative to the primary drug delivery assembly 16.

The receiving element 38 is configured as a user-operable dose setter with a serrated outer surface and connected to the dose setter of the primary drug delivery assembly such that rotation of the receiving element 38 relative to the primary assembly housing is directly transferred to the dose setter 23.

The secondary dose dispensing mechanism of the secondary drug delivery assembly 17 comprises the aforementioned trigger button 50. A projection 51 is provided on the outside of the secondary assembly housing 26 and is normally used to indicate a set dose by indicating a relative rotational position between the projection 51 and the dose knob. The setting element 35 is provided as a cap and is put on the dose knob, wherein the inner surface of the cap has a number of ribs, bump features or the like that engage recesses in the outer surface of the dose knob such that rotation of the setting element 35 is transferred to the dose knob.

The setting element 35 has a cutout 52 which is an axially extending recess, slot or an axially stepped back portion in the sleeve-like distal section of the setting element 35. In other words, at the distal end section of the setting element 35, a circular sector of a distal sleeve section of the setting element 35 has been cut out. The cutout 52 receives the projection 51. The cutout 52 of the setting element 35 extends over a sector of the setting element 35 in circumferential direction. The length of the circular sector which the cutout 52 extends over is chosen such that the setting element 35 is rotatable between a first position in which the projection 51 abuts an inner side wall of the cutout 52 and a second position in which an opposite inner side wall of the cutout 52 abuts against the opposite side of the projection 52. The inner side walls serve in as rotational abutment surfaces. The projection 51 limits the settable dose of the secondary medicament to a maximum dose.

The actuation collar 37 is provided with a second engagement section 53 formed as a toothed rack and is put over the secondary drug delivery assembly 17. A cutout 54 forms a recess in the actuation collar 37, the recess extending from a distal end of the actuation collar 37 in proximal direction. The cutout 54 partly receives the trigger button 50. The cutout 54 in the actuation collar 37 is wider that the width of the trigger button 50. Displacement of the actuation collar 37 in distal direction causes the trigger button 50 to move in the same direction by what the secondary dose dispensing mechanism is actuated and the drive member of the secondary drug delivery assembly 17 urges the cartridge plunger 33 in distal direction.

Figures 4a to 4d show the dose setting and injection process with a drug delivery device 1. The actuator 34 comprises a first engagement section 55 formed on the bar 39 and is rotationally constrained in the housing but axially moveable with respect to the housing in distal direction.

The first engagement section 55 and the second engagement section 53 each have a toothed gear rack for mutual meshed engagement. When the engagement sections 55, 53 are engaged, longitudinal displacement of the actuator 34 in distal direction 3 is transferred to the actuation collar 37 so that the actuation collar 37 is moved distally, too.

In figure 4a, the first engagement section 53 and the first engagement section 55 are not engaged. Accordingly, the actuator 34 is free to move relative to the actuation collar 37 in axial direction. Setting a dose with the primary medicament, requires the patient to rotate the receiving element 38 which causes the dose dial sleeve 19 to move in proximal direction 2. The actuator 34 is connected to the receiving element 38 so that the proximal displacement of the receiving element 38 moves the actuator 34 in proximal direction relative to the secondary engagement section 53 and the housing 7. Accordingly, during setting of a primary medicament dose, the dose setting mechanism of the primary drug delivery assembly 16 and the secondary drug delivery assembly 17 are not mechanically linked which prevents mutual interferences.

When the dose in the primary drug delivery assembly has been set as depicted in Figure 4a, the user rotates the setting element 35 by operating the lever 48 so that the setting element 35 is rotated about the second longitudinal axis 5 from a first position as shown in Figure 4a to a second position as shown in Figure 4b. This rotational movement of the setting element is referred to as the setting movement and is indicated with the curved arrow in Figure 4b.

When the setting element 35 is rotated from the first position into the second position, the setting element 35 transfers rotational movement onto the actuation collar 37. As the actuation collar 37 is arranged coaxially with the setting element 35 it rotates about the same axis 5 as the setting element 35. Rotation of the actuation collar 37 brings its second receiving section 53 into engagement with the first receiving section 55. When the setting element 35 reaches the second position, the first engagement section 55 and the second engagement 53 are brought into engagement. Accordingly, the second position of the setting element 35 corresponds to the engagement between the first engagement section 55 and the second engagement section 53. The setting element 35 has to be rotated entirely from the first into the second position to couple the actuator 34 to the actuation collar 37 so that the drug delivery device is properly operable.

When the secondary dose is set and the actuator 34 is operationally coupled to the actuation collar 37, the patient operates the actuator 34 by displacing the actuator 34 in a longitudinal movement in distal direction 3. This movement is referred to as the actuation movement. The actuation movement is transferred to the actuation collar 37 through the first and second engagement section 55, 53 so that the actuation collar 37 is moved in distal direction and urges the trigger button 50 of the secondary drug delivery assembly 17 in distal direction such that the secondary dose dispensing mechanism is actuated and the set dose of the secondary medicament is expelled under the force of the prestressed biasing member. Accordingly, the setting movement leads to an adjustment of the set dose of the secondary medicament and to the coupling of the actuating movement to the secondary dose dispense mechanism. Only when the setting element is rotated all the way from its first into its second position the set dose of the secondary medicament can be dispensed. In connection with the projection 51 (Figure 3) the variable dose setting mechanism of the secondary drug delivery assembly 17 is turned into a fixed dose setting mechanism.

As the biasing member (torsion spring) of the secondary dose dispensing mechanism relaxes, it rewinds the dose knob into its initial position. This movement is transferred to the setting element 37, which is set from the second position into the first position as shown in Figure 4c. This movement is referred to as the resetting movement indicated with the curved arrow in Figure 4c. When the setting element 37 rotates in the direction opposite to the setting movement, it transfers the rotational movement again onto the actuation collar 37 so that the actuation collar 37 is rotated in the same direction. Thereby, the first engagement section 55 and the second engagement section 53 are disengaged again. The actuator 34 is then again free to move relative to the actuation collar 37 in longitudinal direction of the housing 7 and can be further displaced in distal direction by the user as shown in Figure 4d so that the said dose of the primary medicament is dispensed.

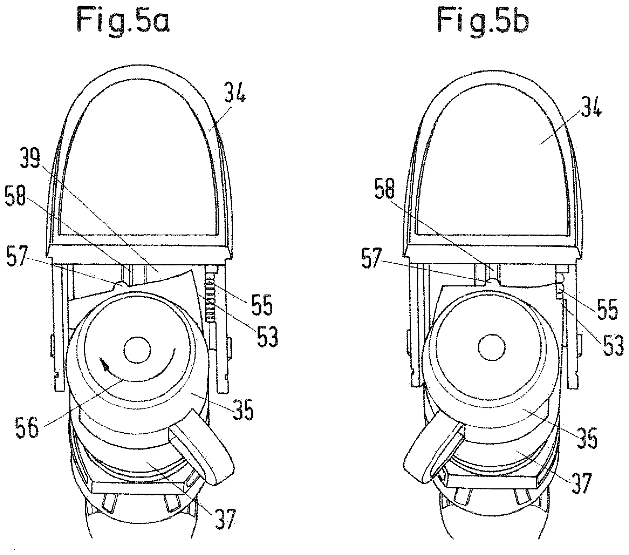

Figures 5a and 5b show the engagement process between the first engagement section 55 and the second engagement section 53. When the setting element 35 is rotated during the setting movement as indicated by the arrow 56, the setting element 35 rotates the actuation collar 37 in the same direction such that first engagement section 55 and the second engagement section 53 engage as displayed in Figure 5b. Each of the engagement sections 55, 53 is provided with a latching portion. The latching portions of the first engagement section 55 and the second engagement section 53 are driven into latching engagement by the setting movement 56. For that purpose, the actuation collar 37 comprises a protruding rib 57 which engages an axially extending groove 58 formed on the bar 39. The protruding rib 57 snaps into the groove 58 with a perceivable sound when the first engagement section 55 and the second engagement section engage 53. Distally oriented flat surfaces of the teeth of the first engagement section 55 and proximally oriented flat surfaces of the teeth of the second engagement section 53 extend perpendicular to the rotational axis of the setting movement 56. Accordingly, these abutment surfaces transmit the actuation movement of the actuator 34 onto the actuation collar 37 but do not prevent the first and second engagement sections 55, 53 from improper disengagement. The rib/groove connection 57/58, however, prevents accidental disengagement between the actuation collar 37 and the actuator 34.

The distally oriented side of the teeth of the second engagement section 53 and the proximally oriented side of the teeth of the first engagement section 55 each have chamfered flanks so that when the actuator 34 moves in a direction opposite the actuation movement, respectively in proximal direction with respect to the actuation collar 37, the chamfered flanks engage and urge the first engagement section 55 and the second engagement section 53 to disengage. On the contrary, the flat surfaces on the respective backside of the chamfered flanks prevent lateral forces that could disengage the first and second engagement section 55, 53.

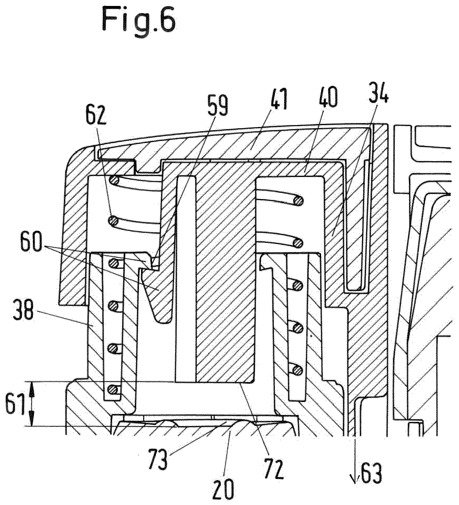

Figure 6 is a sectional view of the proximal end of the actuator 34. The button cap 41 is attached to the proximal end of the actuation section 40. The receiving element 38 is attached to the dose setter of the primary drug delivery assembly. The receiving element 38 is a sleeve-like component with a through-hole 59 through which a projecting of the actuation section 40 extends towards the primary dispense button 20. The distal end of the projection serves as a pressing face 72. The actuator 40 is secured to the receiving element 38 by a snap feature 60 which prevents removal of the actuation section 40 from the receiving element 38. However, the pressing face 72 is initially arranged at a distance with respect to the primary dispense button 20 such that a gap 61 is present between the proximal end of the primary dispense button 20 and the pressing face 72. A biasing member in form of a spring 62 is inserted into the receiving element 38. The spring engages a distal inner surface of the actuation section 40 and urges the actuator 34 in proximal direction with respect to the receiving element 38. The actuator 34 is movable with respect to the receiving element 38 between a first position, in which the pressing face 72 does not contact the primary dispense button 20 and a second position, in which the pressing face 72 makes contact with the primary dispense button 20.

When a dose with the primary medicament has been set and the first and the second engagement sections have been driven into engagement by the setting movement applied to the setting element, the actuator 34 is urged in distal direction by the patient from the first position into the second position with respect to the receiving element 38. In a first phase of the actuation, the actuating movement 63 of the actuator 34 is directly transferred to the actuation collar which engages the trigger button of the secondary drug delivery assembly so that the set dose of the secondary medicament is dispensed. Further displacement of the actuator 34 in distal direction closes the gap 61 so that the pressing face and the proximal surface of the primary dispense button 20 , which serves as a pressure receiving section 73, make contact and the actuation movement 63 of the actuator 34 is transferred to the primary dispense button 20 which activates the primary dose dispense mechanism. This gap 61 causes a delay in the activation of the primary dispense mechanism compared to the secondary dose dispense mechanism. As a result, the set dose of the secondary medicament is dispensed prior to dispense of the primary medicament. The result is a mechanical control which provides for a reliable sequential delivery of the primary medicament and the secondary medicament in one application procedure.

Displacement of the actuation collar in distal direction is limited by the trigger button. When the trigger button has been displaced by the actuation collar to the maximum possible displacement in the first phase of the actuation, resp. delivery sequence, the actuator 34 cannot be displaced further in distal direction. In this situation, the gap 61 is so broad such that the actuation collar reaches the maximum possible distal displacement before the gap 61 is closed. Further displacement of the actuator 34 in distal direction is prevented. The user has to release pressure on the actuator 34 such that under the force of the spring 62 the actuator 34 is moved in proximal direction with respect to the receiving element 38. As a result, the chamfered flanks of the first receiving section 55 and the second receiving section 53 engage and the actuator 34 and the actuation collar 37 are urged to disengage supported by the force induced by the resetting movement. After that, the actuator is free to move in axial direction relative to the actuation collar and the patient may fully displace the actuator 34 in distal direction so that the gap 61 is closed and the actuation movement 63 is transferred to the primary dispense button. The set dose of primary medicament is then dispensed, wherein the set dose of secondary medicament is dispensed prior to the primary medicament.

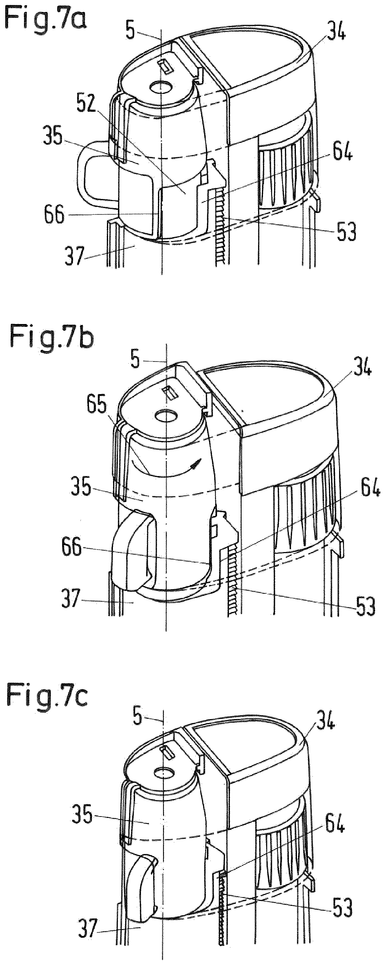

Figures 7a to 7c display the resetting process. In the embodiment of Figures 7a to 7c, the length of the cutout circular sector 52 is larger than a projecting section 64 of the actuation collar 37 in circumferential direction about the second longitudinal axis 5. This allows relative rotational movement between the setting element 35 and the actuation collar between two relative rotational positions. The projecting section 64 is located in the cutout 52. In Figure 7a, the setting element 35 is in its second position after having been rotated from the first position into the second position during the setting movement. In Figure 7a, the setting element 35 is also in a first relative rotational position with respect to the actuation collar 37. When the set dose of secondary medicament is dispensed, the biasing member of the secondary dose setting mechanism rewinds the dose setting element 35 from the second position towards its initial first position (Figure 7b) in the resetting movement 65 about the second longitudinal axis 5. As the length of the cutout circular sector is larger than the projecting section 64 in circumferential direction, the setting member 35 initially rotates relative to the actuation collar 37 into a second relative rotational position with respect to the actuation collar 37. In the second relative rotational position, a rotational abutment surface 66 engages the projecting section 64. Rotational movement of the setting element 35 is then transferred to the actuation collar 37. The setting element 35 continues the resetting movement 65 until the setting element 35 reaches its first position again. During the final travel of the setting element 35, the setting element 35 drives the actuation collar 37 out of its engagement with the actuator 34 and then reaches it first position as shown in Figure 7c. The afore described disengagement function advantageously uses the stored force of the secondary dose setting mechanism to disengage the mechanical connection between the primary dose dispensing mechanism and the secondary dose dispense mechanism.

The setting process may work in a similar way, wherein the process substantially may run backwards from figure 7c to 7a. In circumferential direction opposite to the rotational abutment surface 66 is a further abutment surface (not shown), divided from the rotational abutment surface 66 by the cutout 52. Starting from Figure 7c, the user would rotate the setting element in a setting movement opposite to the resetting movement 65. Due to the cutout 52, the setting element 35 starts to rotate from its first position towards its second position. Initially, the setting member 35 rotates relative to the actuation collar 37. Then, when reaching the first relative rotational position again, the further abutment surface in the cutout 52 engages a respective counter surface on the actuation collar 37, which is located rearwardly (in circumferential direction) to the side of the projection 64 that is engaged by the abutment surface 66 during resetting movement. Upon the engagement, the rotational setting movement of the setting element 35 is transferred to the actuation collar 37 such that the setting element 35 drives the actuation collar 37 in a rotational motion which leads to the engagement of the first engagement section and the second engagement section. Hence, the engagement between the first and the second engagement section 55, 53 can be delayed in the setting process. The cutout 52 is regarding its sectional area dimensioned such that the setting element 35 has to be rotated entirely into its second position to ensure that the setting element 35 is moved into the first relative rotational position and the first and the second engagement section are brought into engagement. In other words, the setting procedure, resp. the setting movement must be completed. Otherwise, the first and the second engagement section do not engage. This safely prevents the activation of the secondary dose dispensing mechanism if the setting element is not moved into the second position properly and ensures that only a predetermined fixed dose can be injected.

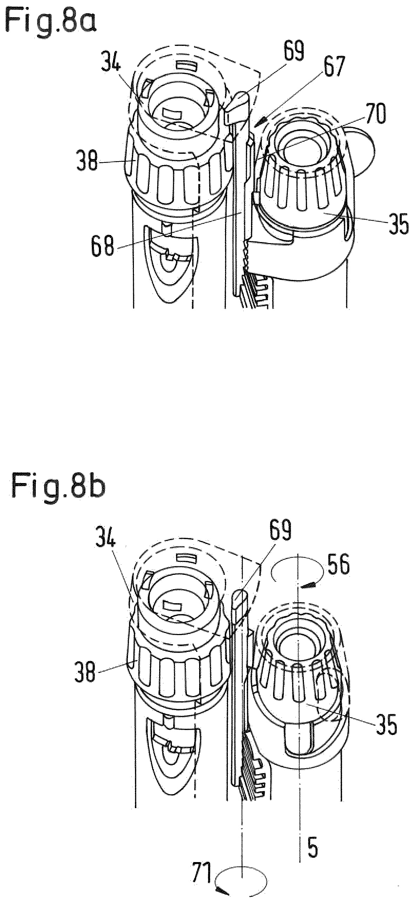

In the embodiment of Figures 8a and 8b, a locking unit 67 comprises a rod 68 with a distal end and a proximal end with a locking pawl 69 and a distal end. The locking unit 67 is rotatably arranged with respect to the housing between a locked position (Figure 8a) and an unlocked position (Figure 8b). In the locked position, the locking pawl 69 engages a free space or a gap between the actuator 34 and the receiving element 38. The locking pawl 69 blocks a movement of the actuator 34 towards the receiving element 38. As a result, the actuator 34 cannot be set from its first into its second position and the primary dose dispensing mechanism cannot be actuated by the actuator 34.

The locking unit 67 is rotatable about a longitudinal axis of the rod 68. A thrust area 70 extends from the rod 68 and is arranged such that when actuation collar 37 is driven into engagement with the actuator 34 by the setting movement 56 of the setting element 35, the actuation collar 37 engages the thrust area 70 and rotates the locking unit 67 about the longitudinal axis of the rod 68 (Figure 8b). By rotation of the locking unit 67 (as indicated by the arrow 71), the locking pawl 69 is swung away from the actuator 34 so that displacement of the actuator 35 in distal direction with respect to the receiving element 38 is no longer prevented. This ensures that a set dose of the primary medicament cannot be dispensed without setting and injecting a fixed dose of the secondary medicament.

### Reference numerals:

- 1: drug delivery device
- 2: proximal end
- 3: distal end
- 4: first longitudinal axis
- 5: second longitudinal axis
- 6: longitudinal axis of housing
- 7: housing
- 8: first half shell
- 9: second half shell
- 10: first side edges
- 11: second side edges
- 12: snap features
- 13: snap features
- 14: first receiving section
- 15: second receiving section
- 16: primary drug delivery assembly
- 17: secondary drug delivery assembly
- 18: primary assembly housing
- 19: dose dial sleeve
- 20: primary dispense button
- 21: cartridge holder
- 22: primary cartridge
- 23: dose setter
- 24: first window
- 25: second window
- 26: secondary assembly housing
- 27: dose knob
- 28: outer screw thread
- 29: cartridge holder
- 30: secondary cartridge
- 31: locking element (auto collar)
- 32: window
- 33: cartridge plunger
- 34: actuator
- 35: setting element
- 36: lever
- 37: actuation collar
- 38: receiving element
- 39: bar
- 40: actuation section
- 41: button cap
- 42: single dispense interface
- 43: cap
- 44: snap feature
- 45: external bump
- 46: recess
- 47: attachment section
- 48: central opening
- 49: closed ring
- 50: trigger button
- 51: projection
- 52: cutout
- 53: second engagement section
- 54: cutout
- 55: first engagement section
- 56: setting movement
- 57: rib
- 58: groove
- 59: through-hole
- 60: snap-feature
- 61: gap
- 62: spring
- 63: actuation movement
- 64: projection section
- 65: resetting movement
- 66: rotational abutment surface
- 67: locking unit
- 68: rod
- 69: locking pawl
- 70: thrust area
- 71: rotation of locking unit
- 72: pressing face
- 73: pressure receiving section

## Claims

1. A housing (7) for a drug delivery device comprising:
a first receiving section (14) for retaining a primary drug delivery assembly (16) with a primary dose setting mechanism to set a dose of a primary medicament and a primary dose dispense mechanism to dispense a set dose of the primary medicament; and a second receiving section (15) for retaining a secondary drug delivery assembly (17) with a secondary dose setting mechanism to set a dose of a secondary medicament and a secondary dose dispense mechanism to dispense a set dose of the secondary medicament;
an actuator (34) arranged such that it is movable along a first longitudinal axis (4) and comprising a first engagement section (55), wherein the actuator (34) is configured to actuate the primary dose dispensing mechanism by an actuation movement (63) such that the primary medicament is delivered;
a lever actuatable setting element (35) located in the second receiving section (15),
**characterized by** an actuation collar (37) comprising a second engagement section (53),
wherein the setting element (35) is configured to be coupled to the secondary dose setting mechanism such that a setting movement (56) of the setting element (35) around a second longitudinal axis (5) from a first position into a second position leads to the setting of a dose in the secondary drug delivery assembly (17) wherein the actuation collar (37) is configured to actuate the secondary dose dispensing mechanism when the actuation movement (63) is transferred to the actuation collar (37) such that the secondary medicament is delivered, and
wherein the setting element (35) drives the actuation collar (37) into engagement with the actuator (34) during the setting movement (56), wherein in the first position of the setting element (35) the actuator (34) is free to move relative to the actuation collar (37) and in the second position of the setting element (35) the actuation collar (37) engages the actuator (34) such that the actuation movement (63) of the actuator (34) is transferred to the actuation collar (37).

2. A housing according to claim 1, wherein the setting element (35) is configured to transfer rotational movement onto the actuation collar (37) over at least a part of the setting movement (56).

3. A housing according to any of the claims 1 or 2, wherein the actuator (34) is rotationally constrained with respect to the housing (7).

4. A housing according to any of the preceding claims, wherein the first engagement section (55) and the second engagement section (53) are configured for mutual meshed engagement.

5. A housing according to any of the preceding claims, wherein the first engagement section (55) and the second engagement section (53) are each connected to a latching portion, wherein the latching portions are configured to be driven into latching engagement when the setting element (35) is moved from the first position into the second position.

6. A housing according to any of the preceding claims, wherein the actuation collar (37) comprises at least one protruding rib (57) configured to engage at least one groove (58) on the actuator (34).

7. A housing according to any of the preceding claims, further comprising a locking element (31) configured to rigidly engage the first and/or the second drug delivery assembly (16, 17) and to be detachably received within the housing (7), wherein the housing (7) comprises at least two housing parts (8, 9) configured to rigidly receive the locking element (31) when the housing parts (8, 9) are assembled.

8. A housing according to any of the preceding claims comprising a receiving element (38) to be attached to a primary dose setting mechanism of the first drug delivery assembly (16), wherein the actuator (34) is movable relative to the receiving element (38) in the axial direction from a first into and a second position during an actuation movement (63) of the actuator (34), wherein the actuator (34) actuates a primary dose dispense mechanism of the primary drug delivery assembly (16) when the actuator (34) is moved into the second position.

9. A housing according to claim 8, wherein a locking unit (67) is switchable between a locked and an unlocked position and arranged such that in the locked position, movement of the actuator (34) from the first into the second position is prevented and in the unlocked position, movement of the actuator (34) from the first into the second position is allowed.

10. A housing according to claim 9, wherein the locking unit (67) is coupled to the setting element (35) such that the setting movement (56) moves the locking unit (67) from the locked position into the unlocked position.

11. A drug delivery device comprising a housing (7) according to any of the preceding claims, wherein the first receiving section (14) retains
a primary drug delivery assembly (16) with a primary dose setting mechanism to set a dose of a primary medicament and a primary dose dispense mechanism to dispense a set dose of the primary medicament through a dispense interface (42), wherein the primary dose dispense mechanism is configured to be actuated by the actuator (34) during the actuation movement (63) of the actuator (34);
wherein the second receiving section (15) retains a secondary drug delivery assembly (17) with a secondary dose setting mechanism to set a dose of a secondary medicament and a secondary dose dispense mechanism to dispense a set dose of the secondary medicament through the dispense interface (42), wherein the setting element (35) is operably coupled to the secondary dose setting mechanism such that the setting movement (56) of the setting element (35) leads to setting of the dose of the secondary medicament and to coupling of the actuation movement (63) to the secondary dispense mechanism.

12. Drug delivery device according to claim 11, wherein the primary dose setting mechanism and the secondary dose setting mechanism are variable dose setting mechanisms.

13. Drug delivery device according to any of the claims 11 to 12, wherein the secondary drug delivery assembly (17) comprises a prestressable biasing member configured to relax and to dispense the dose of the secondary medicament when the secondary dose dispensing mechanism is actuated.

14. Drug delivery device according to any of the claims 11 to 13, wherein the setting element (35) is provided with rotational abutment surfaces (66) limiting the setting movement and thereby rendering the settable dose of the secondary medicament to a fixed dose.

15. Drug delivery device according to any of the claims 11 to 14, wherein the actuator (34) has a pressing face (72) configured to engage a pressure receiving section (73) of the primary dose dispense mechanism and arranged such that by the actuation movement (63) of the actuator (34), a gap (61) between the pressing face (72) and the pressure receiving section (73) is closed and the actuation movement (63) of the actuator (34) is transferred to the primary dose dispense mechanism.

16. Drug delivery device according to any of the claims 11 to 15 comprising at least one cartridge (22, 30) containing a medicament.

## Patentansprüche

1. Gehäuse (7) für eine Medikamenten-Verabreichungsvorrichtung, aufweisend:
einen ersten Aufnahmeabschnitt (14) zum Halten einer Anordnung (16) zur Verabreichung eines primären Medikaments mit einem Mechanismus zum Einstellen einer primären Dosis, um eine Dosis eines primären Medikaments einzustellen, und einem Mechanismus zur Abgabe einer primären Dosis, um eine eingestellte Dosis des primären Medikaments abzugeben, und einen zweiten Aufnahmeabschnitt (15) zum Halten einer Anordnung (17) zur Verabreichung eines sekundären Medikaments mit einem Mechanismus zum Einstellen einer sekundären Dosis, um eine Dosis eines sekundären Medikaments einzustellen, und einem Mechanismus zur Abgabe einer sekundären Dosis, um eine eingestellte Dosis des sekundären Medikaments abzugeben,
einen Aktuator (34), der so angeordnet ist, dass er entlang einer ersten Längsachse (4) beweglich ist, und einen ersten Eingriffsabschnitt (55) aufweist,
wobei der Aktuator (34) dazu ausgestaltet ist, den Mechanismus zur Abgabe einer primären Dosis durch eine Betätigungsbewegung (63) zu betätigen, so dass das primäre Medikament verabreicht wird,
ein durch einen Hebel betätigbares Einstellelement (35), das in dem zweiten Aufnahmeabschnitt (15) angeordnet ist,
**gekennzeichnet durch** einen Betätigungsbund (37), der einen zweiten Eingriffsabschnitt (53) aufweist,
wobei das Einstellelement (35) dazu ausgestaltet ist, an den Mechanismus zum Einstellen einer sekundären Dosis gekoppelt zu werden, so dass eine Einstellbewegung (56) des Einstellelements (35) um eine zweite Längsachse (5) aus einer ersten Position in eine zweite Position zu dem Einstellen einer Dosis in der Anordnung (17) zur Verabreichung eines sekundären Medikaments führt,
wobei der Betätigungsbund (37) dazu ausgestaltet ist, den Mechanismus zur Abgabe einer sekundären Dosis zu betätigen, wenn die Betätigungsbewegung (63) zu dem Betätigungsbund (37) übertragen wird, so dass das sekundäre Medikament verabreicht wird, und
wobei das Einstellelement (35) den Betätigungsbund (37) während der Einstellbewegung (56) in Eingriff mit dem Aktuator (34) treibt, wobei sich der Aktuator (34) in der ersten Position des Einstellelements (35) frei relativ zu dem Betätigungsbund (37) bewegen kann und der Betätigungsbund (37) in der zweiten Position des Einstellelements (35) mit dem Aktuator (34) in Eingriff gelangt, so dass die Betätigungsbewegung (63) des Aktuators (34) auf den Betätigungsbund (37) übertragen wird.

2. Gehäuse nach Anspruch 1, wobei das Einstellelement (35) dazu ausgestaltet ist, eine Drehbewegung auf den Betätigungsbund (37) über zumindest einen Teil der Einstellbewegung (56) zu übertragen.

3. Gehäuse nach einem der Ansprüche 1 oder 2, wobei der Aktuator (34) bezüglich des Gehäuses (7) gegen Drehung gesichert festgehalten ist.

4. Gehäuse nach einem der vorhergehenden Ansprüche, wobei der erste Eingriffsabschnitt (55) und der zweite Eingriffsabschnitt (53) für gegenseitigen kämmenden Eingriff ausgestaltet sind.

5. Gehäuse nach einem der vorhergehenden Ansprüche, wobei der erste Eingriffsabschnitt (55) und der zweite Eingriffsabschnitt (53) jeweils mit einem Rastabschnitt verbunden sind, wobei die Rastabschnitte dazu ausgestaltet sind, in Rasteingriff getrieben zu werden, wenn das Einstellelement (35) aus der ersten Position in die zweite Position bewegt wird.

6. Gehäuse nach einem der vorhergehenden Ansprüche, wobei der Betätigungsbund (37) zumindest eine vorragende Rippe (57) aufweist, die dazu ausgestaltet ist, mit zumindest einer Nut (58) an dem Aktuator (34) in Eingriff zu gelangen.

7. Gehäuse nach einem der vorhergehenden Ansprüche, ferner aufweisend ein Verriegelungselement (31), das dazu ausgestaltet ist, mit der ersten und/oder der zweiten Medikamenten-Verabreichungsanordnung (16, 17) starr in Eingriff zu gelangen und trennbar in dem Gehäuse (7) aufgenommen zu werden, wobei das Gehäuse (7) zumindest zwei Gehäuseteile (8, 9) aufweist, die zur starren Aufnahme des Verriegelungselements (31) ausgestaltet sind, wenn die Gehäuseteile (8, 9) montiert werden.

8. Gehäuse nach einem der vorhergehenden Ansprüche, aufweisend ein an einem Mechanismus zum Einstellen einer primären Dosis der ersten Medikamenten-Verabreichungsanordnung (16) anzubringendes Aufnahmeelement (38), wobei der Aktuator (34) während einer Betätigungsbewegung (63) des Aktuators (34) relativ zu dem Aufnahmeelement (38) in der axialen Richtung aus einer ersten in eine zweite Position beweglich ist, wobei der Aktuator (34) einen Mechanismus zur Abgabe einer primären Dosis der Anordnung (16) zur Verabreichung eines primären Medikaments, wenn der Aktuator (34) in die zweite Position bewegt wird, betätigt.

9. Gehäuse nach Anspruch 8, wobei eine Verriegelungseinheit (67) zwischen einer verriegelten und einer entriegelten Position schaltbar und so angeordnet ist, dass in der verriegelten Position eine Bewegung des Aktuators (34) aus der ersten in die zweite Position verhindert wird, und in der entriegelten Position eine Bewegung des Aktuators (34) aus der ersten in die zweite Position gestattet ist.

10. Gehäuse nach Anspruch 9, wobei die Verriegelungseinheit (67) so an das Einstellelement (35) gekoppelt ist, dass die Verriegelungseinheit (67) durch die Einstellbewegung (56) aus der verriegelten Position in die entriegelte Position bewegt wird.

11. Medikamenten-Verabreichungsvorrichtung, aufweisend ein Gehäuse (7) nach einem der vorhergehenden Ansprüche, wobei der erste Aufnahmeabschnitt (14) eine Anordnung (16) zur Verabreichung eines primären Medikaments mit einem Mechanismus zum Einstellen einer primären Dosis, um eine Dosis eines primären Medikaments einzustellen, und einem Mechanismus zur Abgabe einer primären Dosis, um eine eingestellte Dosis des primären Medikaments durch eine Abgabeschnittstelle (42) abzugeben, hält, wobei der Mechanismus zur Abgabe einer primären Dosis dazu ausgestaltet ist, von dem Aktuator (34) während der Betätigungsbewegung (63) des Aktuators (34) betätigt zu werden,
wobei der zweite Aufnahmeabschnitt (15) eine Anordnung (17) zur Verabreichung eines sekundären Medikaments mit einem Mechanismus zum Einstellen einer sekundären Dosis, um eine Dosis eines sekundären Medikaments einzustellen, und einem Mechanismus zur Abgabe einer sekundären Dosis, um eine eingestellte Dosis des sekundären Medikaments durch die Abgabeschnittstelle (42) abzugeben, hält, wobei das Einstellelement (35) an den Mechanismus zum Einstellen einer sekundären Dosis wirkgekoppelt ist, so dass die Einstellbewegung (56) des Einstellelements (35) zum Einstellen der Dosis des sekundären Medikaments und zum Koppeln der Betätigungsbewegung (63) an den sekundären Abgabemechanismus führt.

12. Medikamenten-Verabreichungsvorrichtung nach Anspruch 11, wobei der Mechanismus zum Einstellen einer primären Dosis und der Mechanismus zum Einstellen einer sekundären Dosis Mechanismen zum Einstellen einer variablen Dosis sind.

13. Medikamenten-Verabreichungsvorrichtung nach einem der Ansprüche 11 bis 12, wobei die Anordnung (17) zur Verabreichung des sekundären Medikaments ein vorspannbares Vorspannglied aufweist, das dazu ausgestaltet ist, sich zu entspannen und die Dosis des sekundären Medikaments abzugeben, wenn der Mechanismus zur Abgabe einer sekundären Dosis betätigt wird.

14. Medikamenten-Verabreichungsvorrichtung nach einem der Ansprüche 11 bis 13, wobei das Einstellelement (35) mit Drehanlageflächen (66) versehen ist, die die Einstellbewegung begrenzen und dadurch aus der einstellbaren Dosis des sekundären Medikaments eine festgelegte Dosis machen.

15. Medikamenten-Verabreichungsvorrichtung nach einem der Ansprüche 11 bis 14, wobei der Aktuator (34) eine Druckfläche (72) hat, die dazu ausgestaltet ist, mit einem Druckaufnahmeabschnitt (73) des Mechanismus zur Abgabe einer primären Dosis in Eingriff zu gelangen, und so angeordnet ist, dass durch die Betätigungsbewegung (63) des Aktuators (34) ein Spalt (61) zwischen der Druckfläche (72) und dem Druckaufnahmeabschnitt (73) geschlossen wird und die Betätigungsbewegung (63) des Aktuators (34) an den Mechanismus zur Abgabe einer primären Dosis übertragen wird.

16. Medikamenten-Verabreichungsvorrichtung nach einem der Ansprüche 11 bis 15, aufweisend zumindest eine Kartusche (22, 30), die ein Medikament enthält.

## Revendications

1. Boîtier (7) pour un dispositif d'administration de médicament, comprenant :
une première section de réception (14) destinée à retenir un ensemble d'administration de médicament primaire (16) avec un mécanisme de réglage de dose primaire pour régler une dose d'un médicament primaire et un mécanisme de distribution de dose primaire pour distribuer une dose réglée du médicament primaire ; et une deuxième section de réception (15) destinée à retenir un ensemble d'administration de médicament secondaire (17) avec un mécanisme de réglage de dose secondaire pour régler une dose d'un médicament secondaire et un mécanisme de distribution de dose secondaire pour distribuer une dose réglée du médicament secondaire ;
un actionneur (34) agencé de manière à pouvoir être déplacé le long d'un premier axe longitudinal (4) et comprenant une première section d'engagement (55), l'actionneur (34) étant configuré pour actionner le mécanisme de distribution de dose primaire par un mouvement d'actionnement (63) de telle sorte que le médicament primaire soit administré ;
un élément de réglage (35) pouvant être actionné par un levier, situé dans la deuxième section de réception (15),
**caractérisé par** un collier d'actionnement (37) comprenant une deuxième section d'engagement (53), l'élément de réglage (35) étant configuré pour être accouplé au mécanisme de réglage de dose secondaire de telle sorte qu'un mouvement de réglage (56) de l'élément de réglage (35) autour d'un deuxième axe longitudinal (5) d'une première position à une deuxième position conduise au réglage d'une dose dans l'ensemble d'administration de médicament secondaire (17), le collier d'actionnement (37) étant configuré pour actionner le mécanisme de distribution de dose secondaire lorsque le mouvement d'actionnement (63) est transféré au collier d'actionnement (37) de telle sorte que le médicament secondaire soit administré, et
l'élément de réglage (35) entraînant le collier d'actionnement (37) en prise avec l'actionneur (34) au cours du mouvement de réglage (56), l'actionneur (34), dans la première position de l'élément de réglage (35), pouvant se déplacer librement par rapport au collier d'actionnement (37), et dans la deuxième position de l'élément de réglage (35), le collier d'actionnement (37) venant en prise avec l'actionneur (34) de telle sorte que le mouvement d'actionnement (63) de l'actionneur (34) soit transféré au collier d'actionnement (37).

2. Boîtier selon la revendication 1, dans lequel l'élément de réglage (35) est configuré pour transférer un mouvement de rotation au collier d'actionnement (37) au cours d'au moins une partie du mouvement de réglage (56).

3. Boîtier selon l'une quelconque des revendications 1 et 2, dans lequel l'actionneur (34) est retenu de manière solidaire en rotation par rapport au boîtier (7).

4. Boîtier selon l'une quelconque des revendications précédentes, dans lequel la première section d'engagement (55) et la deuxième section d'engagement (53) sont configurées pour s'engager l'une dans l'autre par engrènement mutuel.

5. Boîtier selon l'une quelconque des revendications précédentes, dans lequel la première section d'engagement (55) et la deuxième section d'engagement (53) sont chacune connectées à une partie d'enclenchement, les parties d'enclenchement étant configurées pour être entraînées en engagement d'enclenchement lorsque l'élément de réglage (35) est déplacé de la première position à la deuxième position.

6. Boîtier selon l'une quelconque des revendications précédentes, dans lequel le collier d'actionnement (37) comprend au moins une nervure saillante (57) configurée pour venir en prise avec au moins une gorge (58) sur l'actionneur (34).

7. Boîtier selon l'une quelconque des revendications précédentes, comprenant en outre un élément de verrouillage (31) configuré pour venir en prise rigidement avec le premier et/ou le deuxième ensemble d'administration de médicament (16, 17) et pour être reçu de manière détachable à l'intérieur du boîtier (7), le boîtier (7) comprenant au moins deux parties de boîtier (8, 9) configurées pour recevoir rigidement l'élément de verrouillage (31) lorsque les parties de boîtier (8, 9) sont assemblées.

8. Boîtier selon l'une quelconque des revendications précédentes, comprenant un élément de réception (38) destiné à être attaché à un mécanisme de réglage de dose primaire du premier ensemble d'administration de médicament (16), l'actionneur (34) pouvant être déplacé par rapport à l'élément de réception (38) dans la direction axiale d'une première à une deuxième position au cours d'un mouvement d'actionnement (63) de l'actionneur (34), l'actionneur (34) actionnant un mécanisme de distribution de dose primaire de l'ensemble d'administration de médicament primaire (16) lorsque l'actionneur (34) est déplacé dans la deuxième position.

9. Boîtier selon la revendication 8, dans lequel une unité de verrouillage (67) peut être commutée entre une position verrouillée et une position déverrouillée et est disposée de telle sorte que dans la position verrouillée, le mouvement de l'actionneur (34) de la première à la deuxième position soit empêché et que dans la position déverrouillée, le mouvement de l'actionneur (34) de la première à la deuxième position soit permis.

10. Boîtier selon la revendication 9, dans lequel l'unité de verrouillage (67) est accouplée à l'élément de réglage (35) de telle sorte que le mouvement de réglage (56) déplace l'unité de verrouillage (67) de la position verrouillée à la position déverrouillée.

11. Dispositif d'administration de médicament comprenant un boîtier (7) selon l'une quelconque des revendications précédentes, dans lequel la première section de réception (14) retient un ensemble d'administration de médicament primaire (16) avec un mécanisme de réglage de dose primaire pour régler une dose d'un médicament primaire et un mécanisme de distribution de dose primaire pour distribuer une dose réglée du médicament primaire par le biais d'une interface de distribution (42), le mécanisme de distribution de dose primaire étant configuré pour être actionné par l'actionneur (34) au cours du mouvement d'actionnement (63) de l'actionneur (34) ;
dans lequel la deuxième section de réception (15) retient un ensemble d'administration de médicament secondaire (17) avec un mécanisme de réglage de dose secondaire pour régler une dose d'un médicament secondaire et un mécanisme de distribution de dose secondaire pour distribuer une dose réglée du médicament secondaire par le biais de l'interface de distribution (42), l'élément de réglage (35) étant accouplé fonctionnellement au mécanisme de réglage de dose secondaire de telle sorte que le mouvement de réglage (56) de l'élément de réglage (35) conduise au réglage de la dose de médicament secondaire et à l'accouplement du mouvement d'actionnement (63) au mécanisme de distribution secondaire.

12. Dispositif d'administration de médicament selon la revendication 11, dans lequel le mécanisme de réglage de dose primaire et le mécanisme de réglage de dose secondaire sont des mécanismes de réglage de dose variables.

13. Dispositif d'administration de médicament selon l'une quelconque des revendications 11 à 12, dans lequel l'ensemble d'administration de médicament secondaire (17) comprend un organe de sollicitation pouvant être précontraint, configuré pour se relâcher et pour distribuer la dose du médicament secondaire lorsque le mécanisme de distribution de dose secondaire est actionné.

14. Dispositif d'administration de médicament selon l'une quelconque des revendications 11 à 13, dans lequel l'élément de réglage (35) est pourvu de surfaces de butée rotatives (66) limitant le mouvement de réglage et asservissant ainsi la dose réglable du médicament secondaire à une dose fixe.

15. Dispositif d'administration de médicament selon l'une quelconque des revendications 11 à 14, dans lequel l'actionneur (34) présente une face de pressage (72) configurée pour venir en prise avec une section de réception de pression (73) du mécanisme de distribution de dose primaire et prévue de telle sorte que sous l'effet du mouvement d'actionnement (63) de l'actionneur (34), un espace (61) entre la face de pressage (72) et la section de réception de pression (73) soit fermé et que le mouvement d'actionnement (63) de l'actionneur (34) soit transféré au mécanisme de distribution de dose primaire.

16. Dispositif d'administration de médicament selon l'une quelconque des revendications 11 à 15, comprenant au moins une cartouche (22, 30) contenant un médicament.
